# EUROPEAN PATENT SPECIFICATION

(11) **EP 1 572 884 B1**
(45) Date of publication and mention of the grant of the patent: **28.10.2009**
(21) Application number: 02761229.0
(22) Date of filing: 02.08.2002
(51) Int. Cl.: C07K 14/00, A61K 38/00

(54) **DOMINANT NEGATIVE VARIANTS OF METHIONINE AMINOPEPTIDASE 2 (METAP2) AND CLINICAL USES THEREOF**
DOMINANT-NEGATIVE VARIANTEN DER METHIONIN-AMINOPEPTIDASE 2 (METAP2) UND KLINISCHE VERWENDUNGEN DAVON
VARIANTS NEGATIFS DOMINANT DE METHIONINE AMINOPEPTIDASE 2 (METAP2) ET LEURS UTILISATIONS CLINIQUES

(30) Priority: 30.08.2001 US 943123
(43) Date of publication of application: 14.09.2005
(73) Proprietor: Saint Louis University, St. Louis, MO 63103 (US)
(72) Inventor: CHANG, Yie-Hwa, St. Louis, MO 63124 (US)
(74) Representative: Brasnett, Adrian Hugh
(86) International application number: PCT/US2002/024661
(87) International publication number: WO 2003/020881

(56) References cited:
- US-A- 5 888 796
- GRIFFITH E C ET AL: "MOLECULAR RECOGNITION OF ANGIOGENESIS INHIBITORS FUMAGILLIN AND OVALICIN BY METHIONINE AMINOPEPTIDASE 2" PROCEEDINGS OF THE NATIONAL ACADEMY OF SCIENCES OF USA, NATIONAL ACADEMY OF SCIENCE, WASHINGTON, DC, US, vol. 95, December 1998 (1998-12), pages 15183-15188, XP002952598 ISSN: 0027-8424
- LI XUAN ET AL: "Amino-terminal protein processing in Saccharomyces cerevisiae is an essential function that requires two distinct methionine aminopeptidases" PROCEEDINGS OF THE NATIONAL ACADEMY OF SCIENCES OF THE UNITED STATES OF AMERICA, vol. 92, no. 26, 1995, pages 12357-12361, XP002416382 ISSN: 0027-8424
- KLINKENBERG MARCO ET AL: "A dominant negative mutation in Saccharomyces cerevisiae methionine aminopeptidase-1 affects catalysis and interferes with the function of methionine aminopeptidase-2" ARCHIVES OF BIOCHEMISTRY AND BIOPHYSICS, vol. 347, no. 2, 15 November 1997 (1997-11-15), pages 193-200, XP002416383 ISSN: 0003-9861
- LOWTHER W T ET AL: "Escherichia coli methionine aminopeptidase: implications of crystallographic analyses of the native, mutant, and inhibited enzymes for the mechanism of catalysis" BIOCHEMISTRY, AMERICAN CHEMICAL SOCIETY. EASTON, PA, US, vol. 38, no. 24, 15 June 1999 (1999-06-15), pages 7678-7688, XP002320531 ISSN: 0006-2960
- VETRO JOSEPH A ET AL: "Evidence of a dominant negative mutant of yeast methionine aminopeptidase type 2 in Saccharomyces cerevisiae" JOURNAL OF CELLULAR BIOCHEMISTRY, vol. 94, no. 4, 1 March 2005 (2005-03-01), pages 656-668, XP002416384 ISSN: 0730-2312

## Description

### Background of the Invention

### Field of the Invention

The present invention generally relates to inhibitors of type 2 methionine aminopeptidases ("MetAP2"). More specifically, the invention relates to dominant negative variants of MetAP2 and clinical uses thereof, and assays for detecting inhibitors of MetAP2 and effectors of MetAP2.

### Description of Related Art

*N-terminal methionine aminopeptidase activity*. Eukaryotes initiate translation of endogenous cytosolic mRNA with a methionine-bound initiator tRNA (Met-tRNAᵢ^{Met}). As a result, all nascent polypeptides begin with an initiating N-terminal methionine (Metᵢₙᵢₜ). Methionine aminopeptidases ("MetAP", EC 3.4.11.18) are enzymes that cotranslationally remove Metᵢₙᵢₜ from nascent polypeptides when the second residue is small and uncharged (e.g., Met-Ala, -Cys, -Gly, -Pro, -Ser, -Thr, -Val) (Tsunasawa, S., Stewart, J. W., and Sherman, F. (1985) J Biol Chem 260, 5382-91; Flinta, C., Persson, B., Jornvall, H., and von Heijne, G. (1986) Eur J Biochem 154, 193-6; Ben-Bassat, A., Bauer, K., Chang, S. Y., Myambo, K., Boosman, A., and Chang, S. (1987) J Bacteriol 169, 751-7; Huang, S., Elliott, R. C., Liu, P. S., Koduri, R. K., Weickmann, J. L., Lee, J. H., Blair, L. C., Ghosh-Dastidar, P., Bradshaw, R. A., Bryan, K. M. and others (1987) Biochemistry 26, 8242-6; Boissel, J. P., Kasper, T. J., and Bunn, H. F. (1988) J Biol Chem 263, 8443-9; Hirel, P. H., Schmitter, M. J., Dessen, P., Fayat, G., and Blanquet, S. (1989) Proc Natl Acad Sci U S A 86, 8247-51; Moerschell, R. P., Hosokawa, Y., Tsunasawa, S., and Sherman, F. (1990) J Biol Chem 265, 19638-43).

Prokaryotes initiate translation with an N-formylated methionine-bound initiator tRNA (fMet-tRNA_{f}^{Met}) (Adams, J. M. (1968) J Mol Biol 33, 571-89; Housman, D., Gillespie, D., and Lodish, H. F. (1972) J Mol Biol 65, 163-6; Ball, L. A. and Kaesberg, P. (1973) J Mol Biol 79, 531-7). Thus, in the case of prokaryotes, MetAP requires deformylation of fMetᵢₙᵢₜ before the Metᵢₙᵢₜ can be removed (Solbiati, J., Chapman-Smith, A., Miller, J. L., Miller, C. G., and Cronan, J. E. Jr (1999) J Mol Biol 290, 607-14).

Eubacteria possess one type of MetAP (MetAP1) (Ben-Bassat, A., Bauer, K., Chang, S. Y., Myambo, K., Boosman, A., and Chang, S. (1987) J Bacteriol 169, 751-7; Miller, C. G., Strauch, K. L., Kukral, A. M., Miller, J. L., Wingfield, P. T., Mazzei, G. J., Werlen, R. C., Graber, P., and Movva, N. R. (1987) Proc Natl Acad Sci U S A 84, 2718-22; Nakamura, K., Nakamura, A., Takamatsu, H., Yoshikawa, H., and Yamane, K. (1990) J Biochem (Tokyo) 107, 603-7) whereas archaebacteria possess a second type (MetAP2) (Tsunasawa, S., Izu, Y., Miyagi, M., and Kato, I. (1997) J Biochem (Tokyo) 122, 843-50). Although structurally similar, MetAP1 and MetAP2 differ in substrate specificity *in vitro* (Chang, Y. H., Teichert, U., and Smith, J. A. (1992) J Biol Chem 267, 8007-11; Li, X. and Chang, Y. H. (1995) Proc Natl Acad Sci U S A 92, 12357-61; Turk, B. E., Griffith, E. C., Wolf, S., Biemann, K., Chang, Y. H., and Liu, J. O. (1999) Chem Biol 6, 823-33; Walker, K. W. and Bradshaw, R. A. (1999) J Biol Chem 274, 13403-9) and *in vivo*. Interestingly, eukaryotes possess both MetAP1 and MetAP2 (Li and Chang (*supra*); Chang, Y. H., Teichert, U., and Smith, J. A. (92) J Biol Chem 267, 8007-11; Arfin, S. M., Kendall, R. L., Hall, L., Weaver, L. H., Stewart, A. E., Matthews, B. W., and Bradshaw, R. A. (95) Proc Natl Acad Sci U S A 92, 7714-8).

Unlike bacterial MetAPs, eukaryotic MetAPs have an extended N-terminal region. Within this N-terminal region, eukaryotic MetAP2 has a highly charged region, herein called a "translation domain", which contains a single polylysine block (yeast MetAP2) (Li and Chang *supra*) or a polyaspartate block flanked by two polylysine blocks (mammalian MetAP2; p67) (Li and Chang *supra*; Wu, S., Gupta, S., Chatterjee, N., Hileman, R. E., Kinzy, T. G., Denslow, N. D., Merrick, W. C., Chakrabarti, D., Osterman, J. C., and Gupta, N. K. (1993) J Biol Chem 268, 10796-81). These charged N-terminal domains have been proposed to mediate the presumed association of yeast MetAP2 with ribosomes or the association of mammalian MetAP2 with eukaryotic initiation factor-2 (elF-2).

*The role of MetAP2 in cell growth and angiogenesis.* MetAP activity is essential for cellular growth. Deletion of the single *MAP* gene in *E. coli* (Chang, S. Y., McGary, E. C., and Chang, S. (1989) J Bacteriol 171, 4071-2) and *S. typhimurium* (Miller, C. G., Kukral, A. M., Miller, J. L., and Movva, N. R. (1989) J Bacteriol 171, 5215-7) or of both *MAP* genes (*MAP1* and *MAP2*) in yeast (*S. cerevisiae*) is lethal (Li and Chang *supra*). Furthermore, recent evidence suggests that MetAP2 activity in mammalian vascular endothelial cells ("VECs") plays an essential role in blood vessel formation (angiogenesis). Two potent angiogenesis inhibitors, TNP-470, which is a synthetic analog of fumagillin, and ovalicin, were found to selectively target and irreversibly inhibit mammalian MetAP2 in proliferating VECs (Griffith, E. C., Su, Z., Turk, B. E., Chen, S., Chang, Y. H., Wu, Z., Biemann, K., and Liu, J. O. (1997) Chem Biol 4, 461-71; Sin, N., Meng, L., Wang, M. Q., Wen, J. J., Bornmann, W. G., and Crews, C. M. (1997) Proc Natl Acad Sci U S A 94, 6099-103). TNP-470 and ovalicin inhibit MetAP2 by covalently binding to the active site histidine, which corresponds to amino acid position number 231 ("His-231") of human MetAP2 (SEQ ID NO:12) (see Griffith et al., Proc. Natl. Acad. Sci. USA 95:15183-15188, 1998). TNP-470 and ovalicin affect angiogenesis by arresting the endothelial cell cycle in the G1 phase by causing an increase in the activation of p53, which causes a concomitant accumulation of the G1 cyclin-dependent kinase inhibitor p21 (Zhang et al., Proc. Natl. Acad. Sci. USA 97:6427-6432, 2000). Interestingly, ovalicin, TNP-470, fumagillin and analogs thereof have been shown to posses potent immunosuppressive activity as well as anti-angiogenic activity (Turk et al., Bioorg. Med. Chem. 6:1163-1169, 1998), suggesting that MetAP2 activity is necessary for immune cell proliferation.

Angiogenesis is the establishment and growth of new blood vessels and is a major factor in the pathogenesis of many diseases. Those diseases include rheumatoid arthritis, cancer and diabetic retinopathy, among others (Griffith *et al.,* 1998). It well known that angiogenesis is an important and necessary step in tumor growth and metastasis (Folkman, N. Engl. J. Med. 285:1182-1186, 1971). In fact, TNP-470 is currently undergoing clinical trials for treating various forms of cancer, in combination with other chemotherapeutic drugs. Unfortunately, in spite of its promise as a therapeutic, TNP-470 is limited in its use as a sole anti-angiogenesis therapy due to its short half-life in the blood stream and dosage-dependent side effects (see Zhang *et al*., 2000).

*The role of MetAP2 in immune system function.* As mentioned above, the MetAP2-specific inhibitor, ovalicin, is a potent immunosupressive agent, suggesting an important role for MetAP2 in immune system-mediated processes. Interestingly. Denton and coworkers (Denton et al., "TNP-470, an anti-angiogenesis agent, is a potent inhibitor of human CD4+ T cell proliferation," 18th Annual Scientific Meeting of the American Society of Transplantation, May, 1999) have demonstrated that a MetAP2 inhibitor suppresses human CD4+ T cell proliferation and prolongs allograft survival in a rat model of chronic cardiac allograft rejection.

*The role of inhibitors of MetAP2 in antifungal and antimicrosporidial therapy*. Several lines of data suggest that MetAP2 activity is necessary for the growth of pathogenic fungi and microsporidia. Microsporidia are intracellular protozoan parasites that are rapidly emerging as opportunistic pathogens in people suffering from AIDS or other immunocompromising disorders. For example, it is shown that fumagillin and TNP-470 are effective against several microsporidial strains in vitro and in an in vivo animal model (Coyle et al., J. Infect. Dis. 177:515-518, 1998; Didier, Antimicrobial Agents and Chemotherapy 41:1541-1546, 1997). Furthermore, *inhibitors* of MetAP2 have been suggested to have specific antifungal properties ( Cardenas et al., "Antifungal activities of antineoplastic agents: Saccharomyces cerevisiae as a model system to study drug action," Clin. Microbiol. Rev. 12:583-611, 1999) and fumagillin has been demonstrated to be effective against the pathogenic fungus *Nosema* (Ketznelson and Jamieson, Science 150:70-71, 1952).

Klinkenberg, Archives Biochem+Biophys 347(2): 193-200 (1997) describes dominant negative variants of MetAP1 which can suppress the growth of yeast cells.

### Summary of the invention

An object of the invention is the surprising discovery of variants of methionine aminopeptidase 2 ("MetAP2") that have dominant negative activity. Described is a polypeptide that inhibits the peptidase activity of naturally occurring MetAP2. The polypeptide comprises a translation domain or a fragment thereof, which contains one or more polybasic stretches of amino acids. Preferred translation domains include, but are not limited to the polybasic stretch from human MetAP2 (SEQ ID NO:1), mouse MetAP2 (SEQ ID NO:2), rat MetAP2 (SEQ ID NO:15) and yeast METAP2 (SEQ ID NO:3).

Preferably, the variant MetAP2 comprises a full length MetAP2, which has an amino acid substitution at amino acid position His²³¹ of human (SEQ ID NO:12), rat (SEQ ID NO:17) or mouse (SEQ ID NO:13) MetAP2, or at position His¹⁷⁴ of yeast METAP2 (SEQ ID NO:14). More preferrably, the histidine is substituted with a nonconserved amino acid. Yet more preferrably, the histidine is substituted with an alanine.

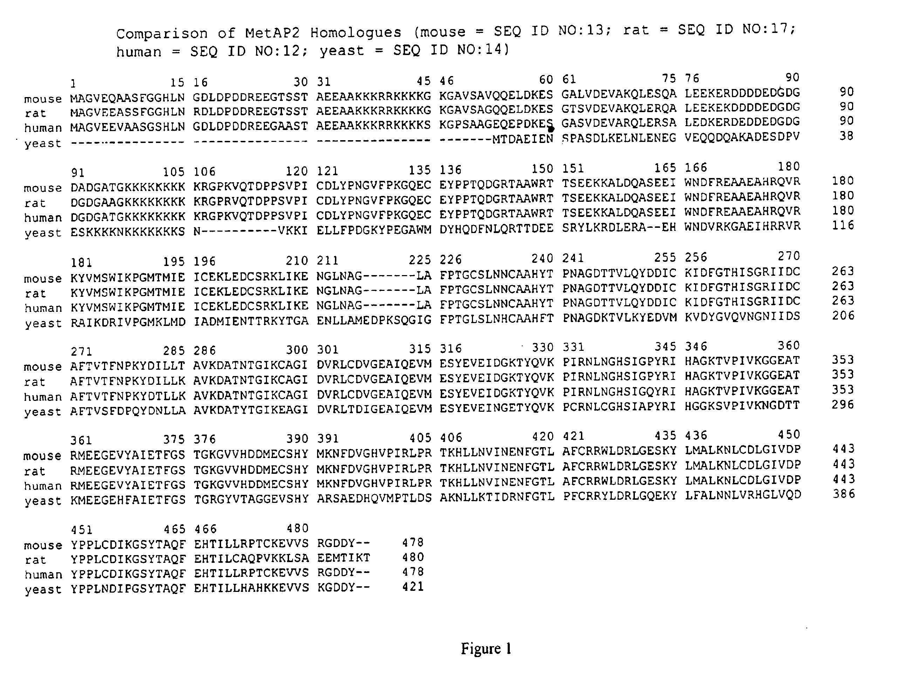

It is also envisioned that polypeptides comprising any translation domain and a histidine substitution at a position analogous to the human His²³¹ residue may function as a MetAP2 dominant negative. Given that human and yeast MetAP2 polypeptides are 46% identical to each other (Figure 1), it is therefore further envisioned that a dominant negative MetAP2 is at least 46% identical to variant human, variant mouse, variant rat, or variant yeast MetAP2 (SEQ ID NO:6, SEQ ID NO:7, SEQ ID NO:16, or SEQ ID NO:8, respectively).

Also described is a polynucleotide that encodes a dominant negative MetAP2 polypeptide, wherein the dominant negative MetAP2 polypeptide comprises a translation domain or a fragment thereof. Preferably the translation domain consists essentially of SEQ ID NO:1, SEQ ID NO:2. SEQ ID NO:3 or SEQ ID NO:15. Alternatively, the polynucleotide encodes a polypeptide of SEQ ID NO:6, SEQ ID NO:7, SEQ ID NO:8 or SEQ ID NO:16. In yet another alternative, the polynucleotide encodes a polypeptide that is at least 46% identical to SEQ ID NO:6, SEQ ID NO:7, SEQ ID NO:8 or SEQ ID NO:16. A more preferred polynucleotide comprises a polynucleotide encoding a variant human MetAP2 with an alanine at position 231 ("H231A"; SEQ ID NO:9), a polynucleotide encoding a variant mouse MetAP2 with a H231A substitution (SEQ ID NO:10), a polynucleotide encoding a variant rat MetAP2 with a H231A substitution (SEQ ID NO:18), or a polynucleotide encoding a variant YEAST MetAP2 with a H174A substitution (SEQ ID NO:11).

Also described is a vector that contains a polynucleotide, which encodes a dominant negative MetAP2 polypeptide, wherein the dominant negative MetAP2 polypeptide comprises a translation domain or a fragment thereof. Preferably, the polybasic stretch consists of SEQ ID NO:1, SEQ ID NO:2. SEQ ID NO:15 or SEQ ID NO:3. Alternatively, the polynucleotide of the vector encodes a polypeptide of SEQ ID NO:6, SEQ ID NO:7, SEQ ID NO:16 or SEQ ID NO:8. In yet another alternative, the polynucleotide of the vector encodes a polypeptide that is at least 46% identical to SEQ ID NO:6, SEQ ID NO:7, SEQ ID NO:16 or SEQ ID NO:8. A more preferred vector contains a polynucleotide comprising SEQ ID NO:9, SEQ ID NO:10, SEQ ID NO:11 or SEQ ID NO:18. The vector may be any plasmid or viral genome. Preferably the vector is a yeast shuttle vector, which is a plasmid that can propagate in bacteria as well as yeast, or an adenovirus, which can infect human cells. It is further envisioned that the variant MetAP2 polynucleotide is operably linked to a promoter that controls or drives the expression of said variant MetAP2. Preferred promoters include regulatable promoters such as for example GAL1, which is active in the presence of galactose and repressed in the presence of glucose, and constitutive promoters such as GPD, which drives the house-keeping gene glyceraldehyde-3-phosphate dehydrogenase, and CMV, which is the cytomegalovirus promoter.

The invention is drawn to the use of variants of MetAP2 with dominant negative activity in methods of treating diseases that are mediated by MetAP2 activity. As discussed above, MetAP2 is involved in cell proliferation, particularly endothelial cell proliferation and immune cell proliferation. Furthermore, it has been shown that inhibition of MetAP2 leads to an increase in p53 activity in cells. Therefore, in another embodiment, the invention is drawn to the use in the treatment of infections, cancers (preferably by inhibiting angiogenesis) and other diseases mediated by insufficient p53 activity, and diseases mediated by the immune system, such as tissue transplant rejections and autoimmune diseases. In vitro methods for inhibiting cell growth comprise contacting a cell with a dominant negative variant MetAP2 polypeptide, as described above, or a polynucleotide that encodes a dominant negative variant of MetAP2. The dominant negative MetAP2 may inhibit the peptidase activity of the wild-type MetAP2 enzyme present in the cell. In a preferred embodiment, the cell is present in a human patient suffering from a cancer or an immune system mediated disorder.

The invention is also drawn to methods of detecting agents that modulate MetAP2 activity. Preferably the method involves the use of a yeast based "synthetic lethal" screen, wherein the expression of the MetAP1 gene may be turned on or off and the wildtype copy of MetAP2 is intact. It is envisioned that negative modulators of MetAP2 activity may cause the arrest of yeast cell growth under conditions in which MetAP1 is not expressed, but permit growth of yeast cells under conditions in which MetAP1 is expressed. It is envisioned that modulators may be compounds that can be directly applied to cells or environmental conditions such as temperature and pH. It is also envisioned that modulators may be polypeptides or polynucleotides. In a preferred embodiment, yeast cells are transformed with polynucleotides, wherein the polynucleotides may encode a dominant negative MetAP2 or another polypeptide that modulates MetAP2 activity.

The invention is also drawn to in vitro methods of treating a cell with agents that modulate MetAP2 activity, wherein the agents are selected according to the yeast "synthetic lethal screen" described above. Preferably, the cell is from a human patient suffering from a cancer or immune system-mediated disease.

The invention is also drawn to a method of detecting, or identifying polypeptides and their encoding polynucleotides that function as downstream effectors of MetAP2. The method of detection may be a yeast based "multicopy suppressor-type" screen wherein a library of genes on a yeast multicopy plasmid, such as a yeast 2 micron plasmid, is transformed into a yeast strain that harbors an active *MAP2* gene (which encodes MetAP2), a dominant negative MetAP2 gene, and a non-functional *map1* gene (the wild-type allele of which encodes MetAP1). It is envisioned that a polynucleotide that encodes a downstream effector of MetAP2 would suppress the dominant negative effect of the dominant negative MetAP2 and allow the yeast cell to proliferate at a greater rate. In a preferred embodiment, the library of genes comprises human polynucleotides.

### Brief Description of Drawings

Figure 1 depicts an alignment of human (SEQ ID NO:12), rat (SEQ ID NO:17), mouse (SEQ ID NO:13) and yeast (SEQ ID NO:14) MetAP2 polypeptides. The alignment was performed using the ClustalW program.

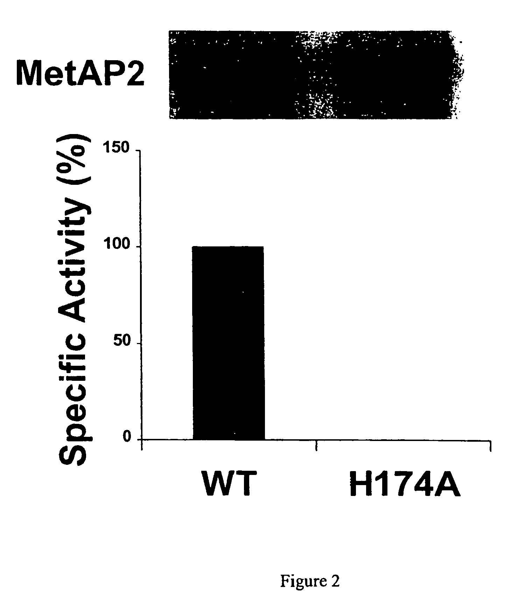

Figure 2 depicts a histogram that demonstrates that H174A-MetAP2 is a dysfunctional catalyst. N-terminal HA-tagged wild-type MetAP2 and MetAP2 (H174A) were immmunopurified from a *map1*Δ strain with mouse anti-hemagglutinin epitope (YPYDVPDYA) (SEQ ID NO:23) monoclonal antibodies. Approximately 2 µg of purified wild-type MetAP2 (WT) and MetAP2 (H174A) was separated on a 10% SDS-PAGE gel and visualized with coomassie blue staining. Specific activity for the wild-type MetAP2 was 11 ± 0.5 U / mg.

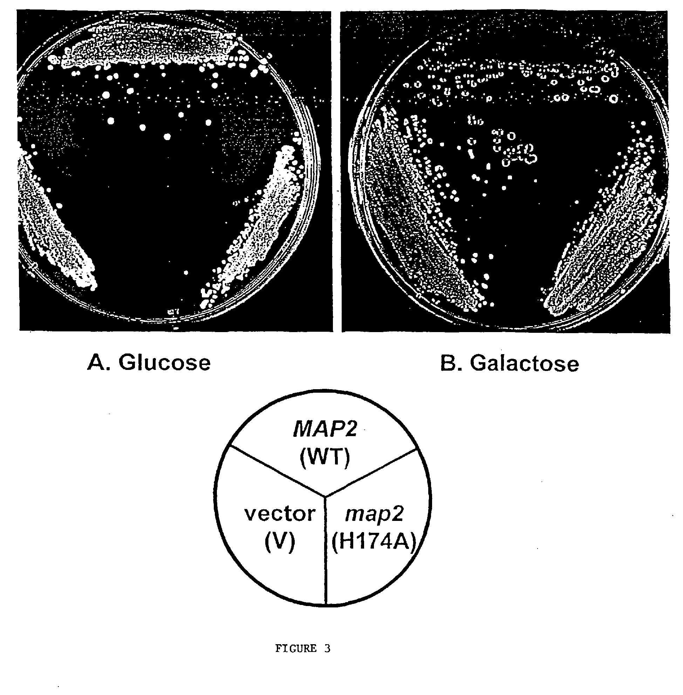

Figure 3 demonstrates that the overexpression of H174A-MetAP2 under GAL1 inhibits the growth of *map1*Δ. A haploid yeast *map1*Δ strain was transformed to leucine prototrophy with p425*GAL1* / *MAP2* wild-type (WT), p425*GAL1* / *map2* (H174A), or p425*GAL1* vector alone (V). Each strain was grown to mid-logarithmic phase at 30°C in minimal synthetic raffinose medium lacking leucine.. Approximately 2 x 10⁵ cells (ABS₆₀₀) were then streaked onto minimal synthetic plates lacking leucine and supplemented with (A) glucose or (B) galactose. Plates were incubated for 4 days at 30°C.

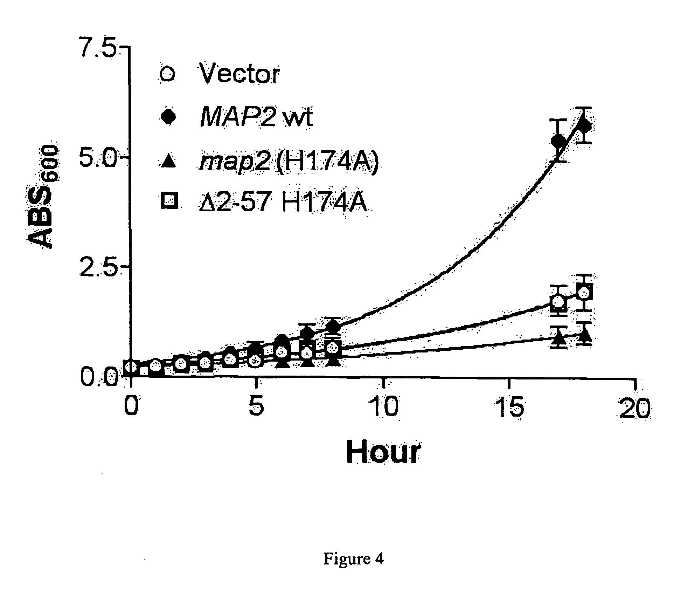

Figure 4 depicts the growth rate of yeast cells harboring various vectors and MetAP2 constructs as determined by a change in optical density.

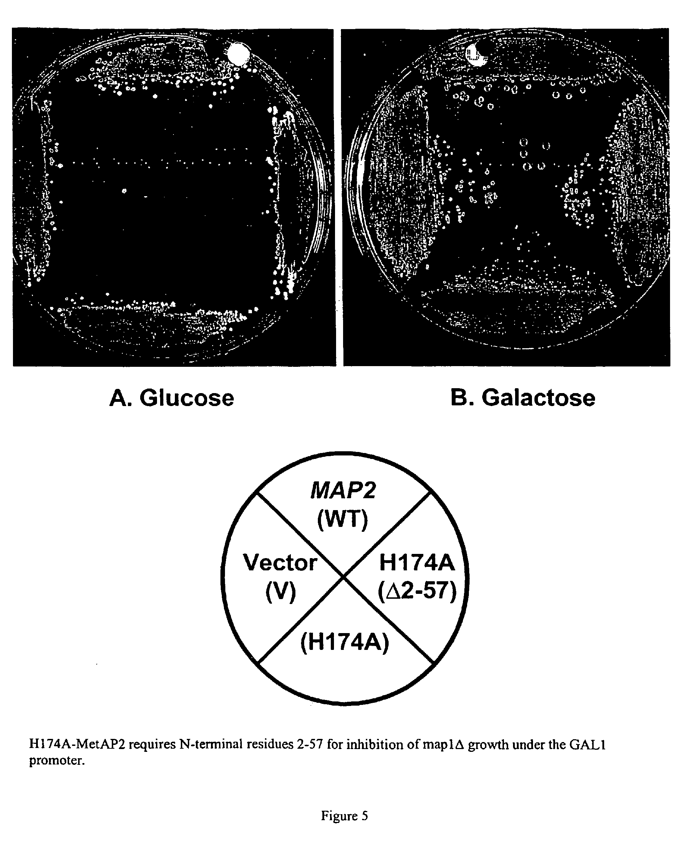

Figure 5 demonstrates that MetAP2 (H174A) requires N-terminal residues 2-57 (which comprises a "translation domain") or inhibition of map1Δ growth under the GAL1 promoter. A haploid yeast *map1*Δ strain was transformed to leucine prototrophy with p425*GAL1* / *MAP2* wild-type (WT), p425*GAL1* / *map2* (H174A), p425*GAL1* / *map2* (Δ2-57 / H174A) or p425*GAL1* vector alone (V). Each strain was grown to mid-logarithmic phase at 30°C in minimal synthetic raffinose medium lacking leucine. Approximately 2 x 10⁵ cells (ABS₆₀₀) were then streaked onto minimal synthetic plates lacking leucine supplemented with (A) glucose or (B) galactose and incubated for 4 days at 30°C.

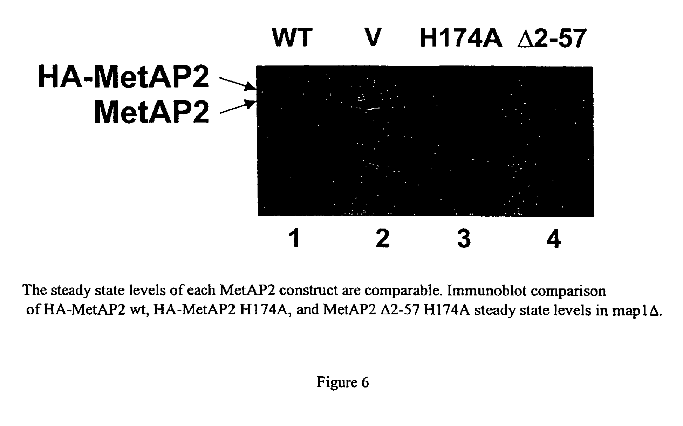

Figure 6 depicts a western blot that demonstrates that the steady state levels of each MetAP2 construct under control of the GAL1 promoter are comparable in *map1*Δ. Each strain was grown to ABS₆₀₀ ∼ 1.0 (25 mL SG/Leu⁻) and a crude extract was obtained. Approximately 1.2 µg of total protein from *map1*Δ expressing wild-type HA-MetAP2 (WT), vector only (V), HA-MetAP2 (H174A), or MetAP2 (Δ2-57 / H174A) was loaded into each lane. Proteins were visualized by western blot using primary rabbit anti-MetAP2 polyclonal antibodies and secondary goat anti-rabbit polyclonal HRP-conjugated anti-bodies. Endogenous wild-type MetAP2 is labeled "MetAP2".

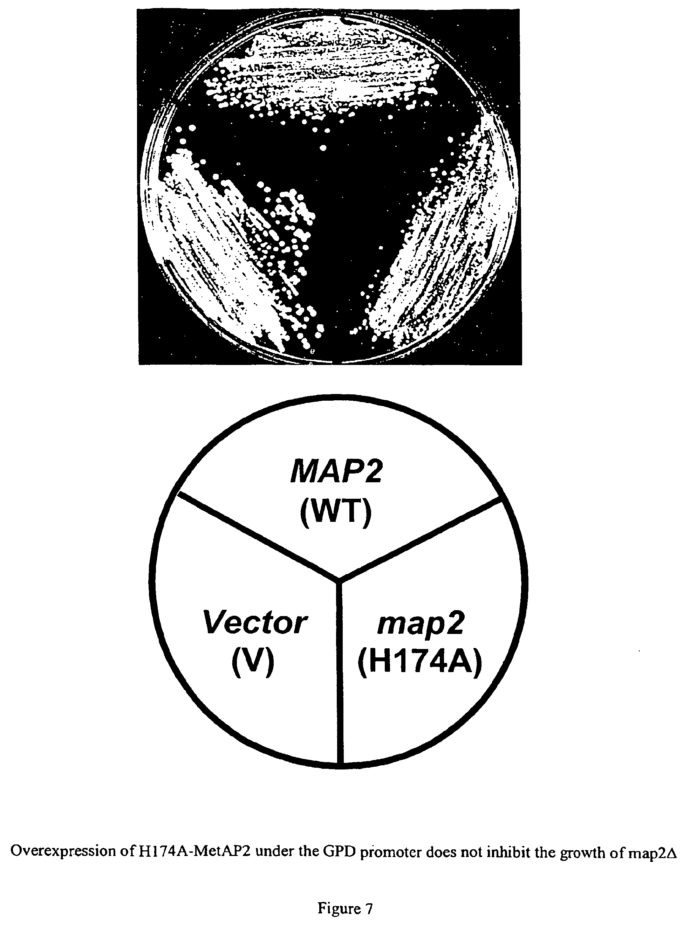

Figure 7 demonstrates that overexpression of MetAP2 (H174A) under the GPD promoter does not affect the growth of *map2*Δ. A haploid yeast *map2*Δ strain was transformed to leucine prototrophy with p425*GPD* / *MAP2* wild-type, p425*GPD* / *map2* (H174A), or p425*GPD* vector alone. Each strain was grown to mid-logarithmic phase at 30°C in minimal synthetic glucose medium lacking leucine. Approximately 2 x 10⁵ cells (ABS₆₀₀) were then streaked onto minimal synthetic glucose plates lacking leucine and incubated for 4 days at 30°C.

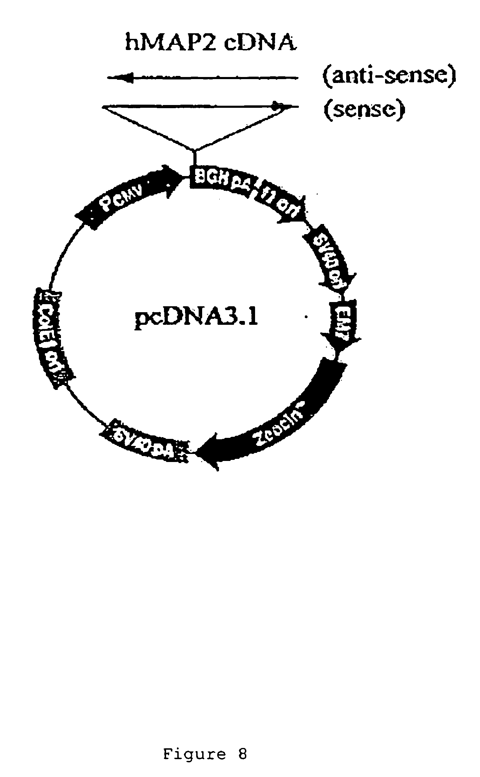

Figure 8 depicts the pcDNA3.1 vector containing either the sense human MetAP2 (*hMAP2*) cDNA or the anti-sense *hMAP2* cDNA.

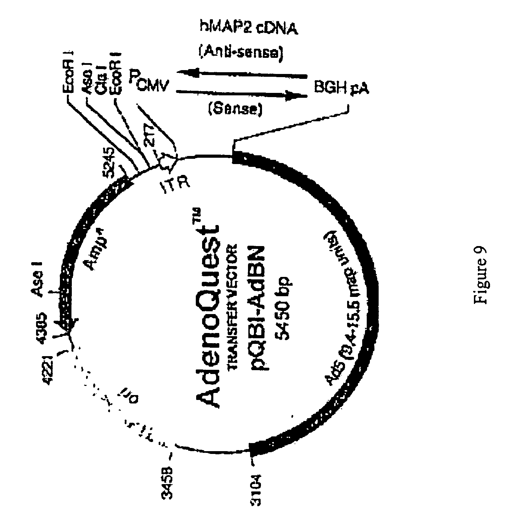

Figure 9 depicts the AdBN vector (adenovirus transfer vector) that contains *hMAP2* cDNA in either the sense or the anti-sense orientation.

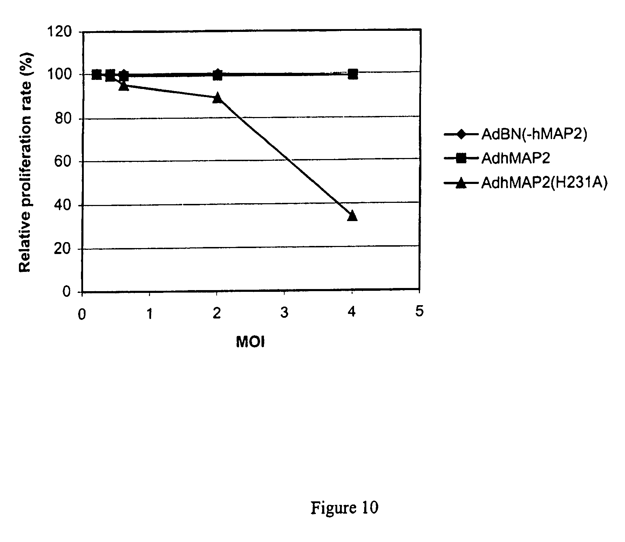

Figure 10 depicts the relative proliferation rate of endothelial cells. Human umbilical vascular endothelial ("HUVE") cells were infected with recombinant adenovirus that harbors the coding sequence of wild-type hMAP2 cDNA (■), dominant negative variant hMAP2(▲), or no hMAP2 (◆) at different multiplicities of infection ("MOI"). Proliferation assays were carried out according to manufacturer's procedures (Cell counting kit-8, Dojindo Molecular Technologies, Inc., MD). Mean values are shown with SD ≤ 15% (n=3).

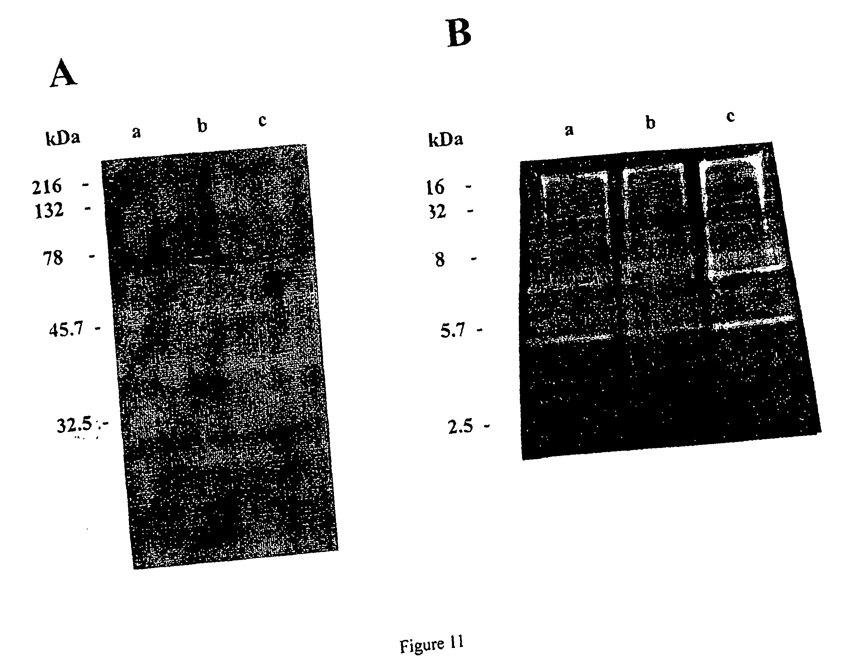

Figure 11 depicts an immunoblot analysis of hMetAP2 expression in the infected HUVE cells. Five (5) µg of total protein obtained from HUVE cells infected with recombinant adenovirus AdBN, AdhMAP2(wt), or AdhMAP2(H231A) at MOI = 4 was loaded onto each lane of two 10% SDS PAGE, respectively. Lane a of panels A and B represents AbBN (empty adenovirus vector), lane b represents AdhMAP2 (wt) and lane c represents AdhMAP2(H231A). One gel was used for inmmunoblot-analysis (A) by transferring the separated proteins onto a nitrocellulose membrane, blotted with anti-HA monoclonal antibodies, followed by incubation with anti-mouse IgG-horse-radish peroxidase conjugate, and the signal was detected by enhanced chemiluminescence (Amersham). The second gel (B) was stained with SYPRO Ruby protein stains according to the manufacturer's procedures (BIO-RAD). Kaleidoscope polypeptide standards from BIO-RAD were used as molecular weight markers.

### Detailed Description of the Invention

Unless defined otherwise, all technical and scientific terms used herein have the same meaning as commonly understood by one of ordinary skill in the art to which this invention belongs. Although any methods or materials similar or equivalent to those described herein can be used in the practice or testing of the present invention, the preferred methods and materials are described. For the purposes of the present invention, the following terms are defined below.

As used herein, the term "translation domain" or "polylysine block" means a peptide domain that mediates the association of any molecule to a ribosome, translation initiation factor or any component of the translational machinery of a cell. Preferably, the translation domain comprises a contiguous stretch of at least three (3) lysine residues. More preferably, the translation domain comprises or consists of the polybasic N-terminal domain of human, rat, mouse or yeast MetAP2 (SEQ ID NO:1, SEQ ID NO:15, SEQ ID NO:2 or SEQ ID NO:3, respectively). More preferably, the translation domain mediates binding to ribosomes or eukaryotic initiation factor 2 ("eIF2").

As used herein, the term "aminopeptidase domain" or "catalytic domain" refers to any C-terminal portion of a MetAP2 polypeptide that does not overlap with the translation domain. The aminopeptidase domain may or may not contain aminopeptidase activity. For example, the aminopeptidase domain of human MetAP2 may span amino acid residues 107 to 478 of SEQ ID NO:12, or any fragment thereof. It is understood that an aminopeptidase domain may be any peptide fragment that does not overlap with a polylysine block, as herein defined, derived from any MetAP2 polypeptide.

The term "native MetAP2 activity" refers to any of the following methionine aminopeptidase activities, which are the cotranslational cleavage of the initiator methionine from nascent polypeptides, promotion of cell proliferation, inhibition of p53 activity, and binding to ribosomes, eIF2 or any component of the translational machinery of the cell.

As used herein , the term "dominant negative MetAP2 activity" or "dominant negative activity" "refers to the inhibition, negation, or diminution of certain particular activities of type 2 methionine aminopeptidase (EC 3.4.11.18). These particular activities of type 2 methionine aminopeptidase include the cleavage of the N-terminal most methionine residue from nascent peptides, the promotion of cell proliferation, angiogenesis and immune system function, and the inhibition of p53 activity. Importantly, these particular activities do not include the regulation of protein synthesis, i.e., binding to ribosomes and binding to eIF2. It is envisioned that upon administering a polypeptide comprising dominant negative MetAP2 activity to a subject or a cell, the naturally occurring MetAP2 activity within that subject or cell is reduced by any amount relative to a control cell that expresses MetAP2. Preferably, the naturally occurring MetAP2 activity is reduced by at least 30%, more preferably by at least 60%, most preferably by 100%.

As used herein, the term "peptide", "polypeptide" or "protein" means a polymer of at least four (4) amino acids linked together via peptide bonds. Said peptide, polypeptide or protein may be covalently modified, wherein the modifications may be any of the art recognized posttranslational modifications, which include for example, methylation, myristoylation, palmitylation, geranylgeranylation or any other lipidation, O-linked glycosylation, N-linked glycosylation or any other glycosylation, glycosylphosphatidylinositol ("GPI") linkage, hydroxylation, phophorylation, polyethylene glycol linkage ("pegylation"), linkage to an albumin molecule ("albumination"), acetylation and ubiquination, among other modifications.

As used herein, the term "polynucleotide" or "nucleic acid" refers to a polymer of four (4) or more nucleotides joined together by phosphodiester bonds. A "nucleotide" is any molecule composed of a nitrogen base, a sugar, and a phosphate group, wherein the sugar is preferably a ribose, deoxyribose or dideoxyribose. The polynucleotide may be single stranded or double stranded molecule. Furthermore, it is to be understood that the use of the term polynucleotide in reference to the encoding of a peptide implies also the non-coding complementary (Crick) strand as well as the coding (Watson) strand.

As used herein, the term "vector" refers to a plasmid, artificial chromosome or virus chromosome used to carry a cloned polynucleotide. Preferred vectors are yeast shuttle vectors, which are plasmids that can be propagated in both yeast and bacteria, adenovectors and baculovirus vectors. Other preferred vectors are gene therapy vectors, including adenovirus.

As used herein, the term "conserved substitution" refers to the interchangeability of amino acid residues having similar side chains. Conservatively substituted amino acids can be grouped according to the chemical properties of their side chains. For example, one grouping of amino acids includes those amino acids that have neutral and hydrophobic side chains (A, V, L, I, P, W, F, and M); another grouping is those amino acids having neutral and polar side chains (G, S, T, Y, C, N, and Q); another grouping is those amino acids having basic side chains (K, R, and H); another grouping is those amino acids having acidic side chains (D and E); another grouping is those amino acids having aliphatic side chains (G, A, V, L, and I); another grouping is those amino acids having aliphatic-hydroxyl side chains (S and T); another grouping is those amino acids having amine-containing side chains (N, Q, K, R, and H); another grouping is those amino acids having aromatic side chains (F, Y, and W); and another grouping is those amino acids having sulfur-containing side chains (C and M). Preferred conservative amino acid substitutions groups are: R-K; E-D, Y-F, L-M; V-I, and Q-H.

As used herein, the term "nonconserved" refers to those amino acids, which are not grouped together as conserved substitutions.

As used herein, the term "agent that modulates MetAP2 activity" refers to any and all polynucleotide, polypeptide molecular compound, molecule, drug, perspective drug, ion, atom or environmental condition that affects in any way the activity of MetAP2. Modulators of MetAP2 activity may completely inhibit, decrease, or increase native MetAP2 activity. Preferably, modulators of MetAP2 activity decrease native MetAP2 activity by at least 30%, more preferably by at least 60%, most preferably by 100%. Preferably, modulators of MetAP2 activity negatively affect cell proliferation.

As used herein, the phrase "diseases mediated by immune system function" or "immunologic diseases" refers to any disease that is affected by the proliferation of immune cells, preferably CD4+ T-cells. Examples of such diseases include at least allograft rejection, which further includes organ transplant rejection, and autoimmune disorders, which further include systemic lupus erythematosus, insulin-dependent diabetes mellitus, rheumatoid arthritis, pemphigus vulgaris, chronic active hepatitis, Sjögren's syndrome, celiac disease, ankylosing spondylitis, delayed type hypersensitivity, glomerulonephritis, polyarteritis nodosa, multiple sclerosis, hemolytic anemia, thrombocytopenic purpura, bullous pemphigoid, myasthenia gravis, Grave's disease, pernicious anemia, insulin-resistant diabetes, rheumatic heart disease, allergic neuritis, allergic encephalomyelitis and autoimmune.

As used herein, the term "effector(s) of MetAP2 function" refers to any and all polypeptides and their encoding polynucleotides that function downstream of MetAP2, especially in the promotion of cell proliferation or progression through the G1 phase of the cell cycle. Preferably, the effector is epistatic in function to any inhibitor of MetAP2 or to a loss of MetAP2 function, wherein epistatic means that the effector can restore a MetAP2 activity in the absence of a functional MetAP2 molecule or in the presence of an inhibitor of MetAP2 activity. For example, a molecule which inhibits p53 activity may be an effector of MetAP2 function.

As used herein, the term "promoter" or "expression regulatory element" refers to a polynucleotide element, which acts as the binding site for RNA polymerase and other transcription activators and is located at or near to the 5' end of a gene. A gene that is "operably linked to a promoter" or "operably linked to an expression regulatory element" is linked in cis to a promoter and its expression is regulated by that promoter. According to the invention, promoters may be constitutive, which refers to promoters that are active under most cellular contexts, or regulatable, which refers to promoters that are either active under specific environmental conditions or active in only specific cell types. Examples of constitutive promoters include CMV, which is active in most mammalian cell types, and GPD, which is active in yeast. Examples of regulatable promoters include tissue-specific promoters, such as "endothelial cell-specific promoters", which include tumor endothelial marker 1 ("TEM1")/endosialin, fetal liver kinase-1 ("Flk-1"), fms-like tyrosine kinase ("Flt-1"), intercellular adhesion molecule-2 ("ICAM-2"), thrombomodulin and von Willebrand factor ("vWF"); "immune cell-specific promoters", which include, for example, CD4, TCR-α, TCR-β, CDR2 or CDR3; and inducible /repressible promoters, such as GAL1, which is active in the presence of galactose and inactive in the presence of glucose.

### Embodiments of the Invention: Theoretical and Experimental Overview

Methionine aminopeptidase type 2 (MetAP2, EC 3.4.11.18) cotranslationally removes N-terminal methionine from nascent polypeptides when the second residue is small and uncharged. MetAP2 consists of two domains: a conserved C-terminal catalytic domain, *i.e*., the "aminopeptidase domain", and an N-terminal polylysine domain predicted to mediate ribosome or eIF2 association, *i.e.,* the "translation domain". According to the present invention, a dominant negative mutant of MetAP2 has been generated which is catalytically inactive against a peptide substrate. In a preferred embodiment, the conserved histidine of the catalytic domain [histidine 231 ("His²³¹") of human (SEQ ID NO:12), mouse (SEQ ID NO:13) or rat (SEQ ID NO:17) MetAP2 , or histidine 174 ("His¹⁷⁴") of yeast MetAP2 (SEQ ID NO:14)] is replaced with another amino acid, preferably a non-conserved amino acid, more preferably an alanine. It is demonstrated herein that overexpression of a variant yeast MetAP2 dominant negative variant (H174A) in a yeast *map1* null strain, which does not express a functional MetAP1 polypeptide, under the strong constitutive GPD promoter was_lethal whereas overexpression under the weaker regulatable *GAL1* promoter significantly inhibited growth (Example 1). These observations suggest that the H174A mutant interferes with wild-type MetAP2 function in a dose-dependent manner. It is herein demonstrated that variant forms of human MetAP2, which lack aminopeptidase function, also have dominant negative activity. Specifically, a variant human MetAP2 comprising a H231A mutation was administered to human vascular endothelial cells in culture and was shown to inhibit vascular endothelial cell growth and endogenous aminopeptidase activity (Example 3). Thus, given that both yeast H171A MetAP2 and human H231A MetAP2 exhibit dominant negative activity, the invention encompasses any and all polypeptides comprising any variant MetAP2 polypeptides that possess dominant negative activity.

His¹⁷⁴ or His²³¹ is strictly conserved in all MetAPs sequenced to date (Li and Chang, PNAS, 1995; Tahirov et al., J. Mol. Biol. 284:101-124, 1998). Previous studies have reported a similar disruption of catalytic function by replacement of the homologous residue in human MetAP2 (H231R) (Griffith *et al*., 1998) or *E. coli* MetAP (H79A) (Lowther et al., Biochemistry, 38:7678-7688, 1999). The homologous residue in human MetAP2, His²³¹, has also been identified as the drug-binding site for two potent angiogenesis inhibitors, TNP-470 and ovalicin (Griffith *et al*., 1998; Liu et al., Science 282:1324-1327, 1998). Thus, the skilled artisan understands that, given the unique molecular structure of histidine, which has a weakly basic imidazole side chain, its position within a beta sheet comprising the active site of the catalytic domain (Liu *et al*., 1998) and its complete conservation in all MetAP2s sequenced to date (Griffith et al., Chemistry and Biology 4:461-471, 1997), this histidine serves an important role in the aminopeptidase function of MetAP2. Therefore, the invention is drawn to variants of MetAP2 which contain any amino acid except histidine at the position corresponding to residue number 231 in human, mouse or rat MetAP2 or residue number 171 in yeast MetAP2. It is further envisioned that any variant form of MetAP2 which abolishes the active site pocket, abolishes fumagillin binding sites and/or amino acids involved in the coordination of a cobalt ion, is also encompassed by the invention. Those amino acids that contact fumagillin are, according to the numbering system of human MetAP2 (SEQ ID NO:12), Phe²¹⁹, Leu³²⁸, Ile³³⁸, His³³⁹, Tyr⁴⁴⁴ and Leu⁴⁴⁷. Those amino acids that are involved in the coordination of Co²⁺ ions are, according to the numbering system of SEQ ID NO:12, Asp²⁵¹, Asp²⁶², His³³¹, Glu³⁶⁴ and Glu⁴⁵⁹. It is envisioned that amino acid substitutions at any of the positions listed above, or in analogous positions of non-human MetAP2 homologues, would give rise to a dominant negative phenotype and therefore must be considered embodiments of the present invention. Preferred dominant negative variants of MetAP2 are depicted as SEQ ID NO:9, SEQ ID NO:10, SEQ ID NO:11 and SEQ ID NO:16.

The inventors herein further demonstrate that the overexpression of truncated MetAP2 (H174A) that lacks the polylysine block ("translation domain"), which comprises amino acids 2-57 of SEQ ID NO:8 and SEQ ID NO:14, had no effect on the growth rate of a *map1* null yeast strain when expressed under either the *GAL1* promoter or the *GPD* promoter. This observation demonstrates that the presence of a polylysine block is necessary to confer dominant negative MetAP2 activity to a polypeptide. It is therefore envisioned that the translation domain from any MetAP2, preferably a human, mouse, rat or yeast polylysine block (SEQ ID NO:1, SEQ ID NO:2, SEQ ID NO:15 or SEQ ID NO:3, respectively), or a fragment thereof may function as a dominant negative MetAP2. Given this important discovery of the role of the translation domain in dominant negative MetAP2 activity, an embodiment of the invention is drawn to polypeptides that comprise a translation domain or fragment thereof, wherein the polypeptide has dominant negative MetAP2 activity and wherein the fragment of said translation domain is not less than three (3) amino acids long.

While this invention is not bound by any theories, a plausible explanation for the dominant negative activity of the polypeptide variants of this invention is that the variants compete with wild-type MetAP2 for association with ribosomes, eIF-2 or other elements of the translation machinery of a cell. It has been assumed that MetAPs are associated with the translational machinery because N-terminal methionine is cotranslationally removed from nascent polypeptides that are approximately 15-20 amino acids in length (Yoshida et al., Proc. Natl. Acad. Sci. USA 67:1600-1607, 1970). Thus, overexpression of the catalytically inactive variant form of MetAP2 may lead to the disruption of cellular MetAP2 activity by displacing functional wild-type MetAP2 from the translational machinery and precluding the cotranslational removal of N-terminal methionine from nascent polypeptides. This is consistent with the apparent dose-dependent effect of the yeast H174A variant in *map1*Δ yeast strains and the requirement for the N-terminal domain, which comprises a polylysine block.

Polylysine blocks have been found in several proteins, including eukaryotic initiation factor 2β (eIF-2β) (Pathak et al., Cell 54:633-639, 1988) and N-myristoyltransferase (Glover et al., J. Biol. Chem. 272:28680-28689, 1997). In most cases, these blocks, together with adjacent acidic residues, are believed to be responsible for protein-protein and protein-nucleic acid interactions, and facilitate the activity of certain factors in protein synthesis. It is worth noting that the polylysine region of human myristoyltransferase was reported to likely play an important role in its ribosomal association (Glover *et al.*, 1997).

For the purposes of this invention, it is important to note that naturally occurring human and mouse MetAP2 polypeptides share 96% sequence identity, whereas naturally occurring yeast MetAP2 is 46% identical to naturally occurring human MetAP2. Figure 1 depicts the alignment of naturally occurring (wild-type) human (SEQ ID NO:12), mouse (SEQ ID NO:13), rat (SEQ ID NO:17) and yeast MetAP2 (SEQ ID NO:14). The alignment was performed using the art recognized ClustalW Multiple Sequence Alignment protocol. Sequence identity was determined by dividing the number of shared identical amino acids by the total number of amino acids in the polypeptide. Therefore, given that the object of the invention is a dominant negative variant of MetAP2, and that the yeast, human, rat and mouse variants are disclosed, it is envisioned that an embodiment of the invention is directed to a dominant negative variant of MetAP2 is at least 46% identical to the human variant sequence of SEQ ID NO:6.

### Embodiments of the Invention: dnvMetAP2 Polypeptides

The present invention is directed to variant type 2 methionine aminopeptidases which selectively inactivate the methionine cleavage activity of naturally occurring type 2 methionine aminopeptidase ("MetAP2") without substantially affecting the translational machinery binding activity of MetAP2. Such dominant negative variants of MetAP2 ("dnvMetAP2") comprise, preferably, a translation domain and an inactive aminopeptidase or catalytic domain. dnvMetAP2s inhibit the cell proliferation-promoting effect of wild-type MetAP2 and are therefore useful for inhibiting, for example, angiogenesis, immune cell proliferation, growth of microsporidia, growth of tumors and the growth of fungi *in vivo* or *in vitro*.

It is envisioned that the dnvMetAP2 polypeptide comprises a translation domain or a fragment thereof, which confers translational machinery binding activity, from any one of naturally occurring MetAP2 polypeptides, either known or currently unknown. Examples of polylysine blocks include those derived from human (SEQ ID NO:1), mouse (SEQ ID NO:2), rat (SEQ ID NO:15) and yeast (SEQ ID NO:3) MetAP2. In another embodiment, the dnvMetAP2 further comprises a aminopeptidase domain or fragment thereof derived from any naturally occurring MetAP2. In a preferred embodiment, the dnvMetAP2 comprises a polypeptide of SEQ ID NO:6, 7, 8 or 16, which corresponds to variant forms of human, mouse, yeast or rat MetAP2, respectively, wherein key amino acid substitutions that eliminate aminopeptidase activity while preserving translation machinery binding are present. Those key substitutions are described above and in the sequence listing, which is herein incorporated by reference, as SEQ ID NO:6, 7, 8 and 16. In a more preferred embodiment, the dnvMetAP2 comprises a non-conserved amino acid substitution for histidine at position 231 of human, mouse or rat MetAP2 or at position 174 of yeast MetAP2.

It is further envisioned that chimeric forms of dnvMetAP2 fall within the scope of this invention, wherein a polylysine block from one MetAP2 homolog is spliced to an aminopeptidase domain of one or more other MetAP2 homologs. A preferred chimeric dnvMetAP2 is a least 46% identical to a human dnvMetAP2 (SEQ ID NO:6) at the amino acid level. Chimeric dnvMetAP2 polypeptides can be made according to any method that is well known in the art. Comparisons of dnvMetAP2 amino acid sequences may be made using alignment methods which are designed to align regions according to similar predicted tertiary structures. An example is the Clustal method (Higgins et al, Cabios 8:189-191, 1992) of multiple sequence alignment in the Lasergene biocomputing software (DNASTAR, INC, Madison, WI). In this method, multiple alignments are carried out in a progressive manner, in which larger and larger alignment groups are assembled using similarity scores calculated from a series of pairwise alignments. Optimal sequence alignments are obtained by finding the maximum alignment score, which is the average of all scores between the separate residues in the alignment, determined from a residue weight table representing the probability of a given amino acid change occurring in two related proteins over a given evolutionary interval. Penalties for opening and lengthening gaps in the alignment contribute to the score. The default parameters used with this program are as follows: gap penalty for multiple alignment = 10; gap length penalty for multiple alignment = 10; k-tuple value in pairwise alignment = 1; gap penalty in pairwise alignment = 3; window value in pairwise alignment = 5; diagonals saved in pairwise alignment = 5. The residue weight table used for that alignment program is PAM250 (Dayhoff et al., in Atlas of Protein Sequence and Structure, Dayhoff, Ed., NBRF, Washington, Vol. 5, suppl. 3, p. 345, 1978).

It is believed that the dnvMetAP2s need not comprise the exact amino acid sequence as depicted in the Sequence Listing or Figure 1 in order to retain the ability to inhibit naturally occurring MetAP2 activity. Rather, conservative amino acid substitutions in the dnvMetAP2s are within the scope of the present invention. Conservative amino acid substitutions refer to the interchangeability of residues having similar side chains. Conservatively substituted amino acids can be grouped according to the chemical properties of their side chains. For example, one grouping of amino acids includes those amino acids have neutral and hydrophobic side chains (A, V, L, I, P, W, F, and M); another grouping is those amino acids having neutral and polar side chains (G, S, T, Y, C, N, and Q); another grouping is those amino acids having basic side chains (K, R, and H); another grouping is those amino acids having acidic side chains (D and E); another grouping is those amino acids having aliphatic side chains (G, A, V, L, and I); another grouping is those amino acids having aliphatic-hydroxyl side chains (S and T); another grouping is those amino acids having amine-containing side chains (N, Q, K, R, and H); another grouping is those amino acids having aromatic side chains (F, Y, and W); and another grouping is those amino acids having sulfur-containing side chains (C and M). Preferred conservative amino acid substitutions groups are: R-K; E-D, Y-F, L-M; V-I, and Q-H.

As used herein, the dnvMetAP2s of the present invention can also include modifications of the sequences identified herein, including sequences in which one or more amino acids have been inserted, deleted or replaced with a different amino acid or a modified or unusual amino acid, as well as modifications such as glycosylation or phosphorylation of one or more amino acids so long as the dnvMetAP2 containing the modified sequence retains the ability to inhibit naturally occurring MetAP2 activity. Amino acid(s) can be added to or removed from the N-terminus, C-terminus or within the amino acid sequence, provided the translational machine binding activity is retained, which is believed to be required for aminopeptidase inhibiting activity. Thus, the sequences set forth in SEQ ID NOS:1-3 and 15, which are sequences of the full length translation domains, are believed to be the minimum domain required for activity of these dnvMetAP2s.

In another embodiment of the invention, it is envisioned that the dnvMetAP2 polypeptides further comprise a transit peptide, preferably fused in frame at the N-terminus of the dnvMetAP2. The transit peptide comprises a hydrophobic or cationic/amphipathic sequence which enables the peptide to cross the plasma membranes of cells. Hydrophobic sequences may comprise portions of the membrane permeable sequences of Karposi FGF, Grb2 or integrin β3, the fusion sequence of HIV-1 gb41 or the signal sequence of *Caiiman croc*. Ig(v) light chain. Amphipathic/cationic sequences may comprise portions of influenza hemagglutinin subunit, antennapedia third helix, HIV-1 Tat, HSV transcription factor, galanin/mastoparin fusion or synthetic analogs thereof. Transit peptides are well known in the art and are well described in Gariepy and Kawamura, Trends Biotech. 19:21-28, 2001. The preferred transit peptide comprises an HIV-1 Tat sequence of SEQ ID NO:19 (Gly-Arg-Lys-Lys-Arg-Arg-Gln-Arg).

A preferred dnvMetAP2 according to the present invention is prepared in pure form by recombinant DNA technology. By "pure form" or "purified form" or "substantially purified form" it is meant that a dnvMetAP2 composition is substantially free of other proteins which are not the dnvMetAP2. Preferably, a substantially purified dnvMetAP2 composition comprises at least about 50 percent dnvMetAP2 on a molar basis compared to total proteins or other macromolecular species present. More preferably, a substantially purified dnvMetAP2 composition will comprise at least about 80 to about 90 mole percent of the total protein or other macromolecular species present and still more preferably, at least about 95 mole percent or greater.

A recombinant dnvMetAP2 may be made by expressing a DNA sequence encoding the dnvMetAP2 in a suitable transformed host cell. Using methods well known in the art, the DNA encoding the dnvMetAP2 may be linked to an expression vector and transformed into a host cell, and conditions established that are suitable for expression of the dnvMetAP2 by the transformed cell.

Any suitable expression vector may be employed to produce a recombinant dnvMetAP2 such as, for example, the mammalian expression vector pCB6 (Brewer, Meth Cell Biol 43: 233-245, 1994) or the *E. coli* pET expression vectors, specifically, pET-30a (Studier et al., Methods Enzymol 185: 60-89, 1990). Other suitable expression vectors for expression in mammalian and bacterial cells are known in the art as are expression vectors for use in yeast or insect cells. Baculovirus expression systems can also be employed.

A number of cell types may be suitable as host cells for expression of a recombinant dnvMetAP2. Mammalian host cells include, but are not limited to, monkey COS cells, Chinese Hamster Ovary (CHO) cells, human kidney 293 cells, human epidermal A431 cells, human Colo 205 cells, 3T3 cells, CV-1 cells, other transformed primate cell lines, normal diploid cells, cell strains derived from *in vitro* culture of primary tissue, primary explants, HeLa cells, mouse L cells, BHK, HL-60, U937, HaK and Jurkat cells. Yeast strains that may act as suitable host cells include *Saccharomyces cerevisiae, Schizosaccharomyces pombe, Kluyveromyces* strains, *Candida, Pichia* and any other yeast strain capable of expressing heterologous proteins. Host bacterial strains include *Escherichia coli, Bacillus subtilis, Salmonella typhimurium* and any other bacterial strain capable of expressing heterologous proteins. If the polypeptide is made in yeast or bacteria, it may be necessary to modify the polypeptide, for example, by phosphorylation or glycosylation of the appropriate sites using known chemical or enzymatic methods, to obtain a biologically active polypeptide.

The dnvMetAP2s of the present invention can also be expressed in transgenic plants (see, for example, U.S. Patent No. 5,679,880) or transgenic animals such as, for example, cows, goats, pigs, or sheep whose somatic or germ cells contain a nucleotide sequence encoding the dnvMetAP2.

The expressed dnvMetAP2 can be purified using known purification procedures, such as gel filtration and ion exchange chromatography. Purification may also include affinity chromatography using an agent that will specifically bind the polypeptide, such as a polyclonal or monoclonal antibody raised against the dnvMetAP2 or fragment thereof. Other affinity resins typically used in protein purification may also be used such as concanavalin A-agarose, HEPARIN-TOYOPEARL^{®} or CIBACROM BLUE 3GA SEPHAROSE^{®}. Purification of the dnvMetAP2 may also include one or more steps involving hydrophobic interaction chromatography using such resins as phenyl ether, butyl ether, or propyl ether.

It is also contemplated that the dnvMetAP2 may be expressed as a fusion protein to facilitate purification. Such fusion proteins, for example, include the dnvMetAP2 amino acid sequence fused to a histidine tag, as well as the dnvMetAP2 amino acid sequence fused to the amino acid sequence of maltose binding protein (MBP), glutathione-S-transferase (GST) or thioredoxin (TRX). Similarly, the invention dnvMetAP2 can be tagged with a heterologous epitope, such as a FLAG^{®} epitope or a myc epitope, and subsequently purified by immunoaffinity chromatography using an antibody that specifically binds the epitope. Kits for expression and purification of such fusion proteins and tagged proteins are commercially available.

The recombinant dnvMetAP2s may also be prepared under reducing conditions. In such instances refolding and renaturation can be accomplished using one of the agents noted above that is known to promote dissociation/association of proteins. For example, the dnvMetAP2 polypeptide may be incubated with dithiothreitol followed by incubation with oxidized glutathione disodium salt followed by incubation with a buffer containing a refolding agent such as urea.

The dnvMetAP2s of the present invention may also be produced by chemical synthesis using methods known to those skilled in the art.

### Embodiments of the Invention: dnvMetAP2 Polynucleotides & Vectors

The present invention also encompasses isolated polynucleotides comprising nucleotide sequences that encode any of the dnvMetAP2s described herein. As used herein, a polynucleotide includes DNA and/or RNA and thus the nucleotide sequences recited in the Sequence Listing as DNA sequences also include the identical RNA sequences with uracil substituted for thymine bases. Preferred polynucleotides encode a dnvMetAP2 comprising an alanine substitution for His231 (H231A in mammalian MetAP2) or for His174 (H174A in yeast MetAP2). Those polynucleotides comprise SEQ ID NO:9, SEQ ID NO:10, SEQ ID NO:11 and SEQ ID NO:18, which encode human, mouse, yeast and rat dnvMetAP2s, respectively.

The present invention also encompasses vectors comprising an expression regulatory element operably linked to any of the dnvMetAP2-encoding nucleotide sequences included within the scope of the invention. This invention also includes host cells, of any variety, that have been transformed with such vectors. Expression regulatory elements are defined above. Vectors which may be used in this invention include vectors that are useful as gene delivery systems for gene therapy, mammalian expression plasmids, bacterial plasmids, yeast shuttle vectors, human artificial chromosomes, yeast artificial chromosomes and cosmids. Gene delivery systems for gene therapy include, for example, retroviruses, adenovirus, adeno-associated viruses, lentiviruses, herpes simplex virus, vaccinia virus, human cytomegalovirus. Epstein-Barr virus, negative-strand viruses and hybrid viral vector systems. A preferred gene therapy vector is adenovirus. For a comprehensive review of gene delivery systems for gene therapy, see Romano et al., Stem Cells 18:19-39, 2000. Also preferred are yeast shuttle vectors useful in yeast-based multicopy suppression screens and synthetic lethal screens. For a comprehensive review on yeast shuttle vectors, see Methods in Yeast Genetics: A Cold Spring Harbor Laboratory Course Manual (2000 Edition) by Dan Burke, Dean Dawson and Tim Steams, CSHL Press, 2000.

In yet another embodiment, a polynucleotide which specifically hybridizes to a dnvMetAP2-encoding polynucleotide or to its complement is provided. Specific hybridization is defined herein as the formation of hybrids between a polynucleotide, including oligonucleotides, and a specific reference polynucleotide (e.g., a polynucleotide comprising a nucleotide sequence complementary to a nucleotide sequence encoding a dnvMetAP2) wherein the polynucleotide preferentially hybridizes to the specific dnvMetAP2 over other aminopeptidases. Specific hybridization is preferably done under high stringency conditions which, as well understood by those skilled in the art, can readily be determined by adjusting several factors during hybridization and during the washing procedure, including temperature, ionic strength, length of hybridization or washing times, and concentration of formamide (see for example, Sambrook, Fritsch and Maniatis., Molecular Cloning: a Laboratory Manual, 2d Ed., Vols. 1-3, Cold Spring Harbor Laboratory Press, Plainview N.Y. 11803, 1989)

### Embodiments of the Invention: Pharmaceutical Compositions & Therapeutics

The dnvMetAP2s of the present invention would be expected to inhibit the growth of microsporidial cells, some bacterial cells, tumor cells and T-cells, as well as fungal cells and endothelial cells as herein shown in Examples 1 and 3 herein. As discussed in the Background of the Invention, inhibitors of MetAP2 have been shown to inhibit the growth of endothelial cells, tumor cells, CD4+ T-cells, microsporidia and some fungi. Therefore, the invention dnvMetAP2s would be expected to also have such an effect on the same and similar cell types.

The present invention also includes therapeutic or pharmaceutical compositions comprising a dnvMetAP2 of the present invention in an effective amount for inhibiting the growth of vascular endothelial cells, immune system cells, microsporidia, fungal cells or bacterial cells in patients suffering from cancer, a disease mediated by the immune system or an opportunistic infection, and a method comprising administering a therapeutically effective amount of the dnvMetAP2 to a cell *ex vivo* or *in vivo*. The compositions and methods of the present invention are preferably useful for treating a cancer by inhibiting or reversing the effects of angiogenesis, whereby angiogenesis is the establishment of a blood supply to a tumor, and for treating diseases mediated by immune system function, as defined above.

Whether the dnvMetAP2s of the present invention would be effective in the inhibition of growth of a particular cell or tissue type can be readily determined by one skilled in the art using any of a variety of assays known in the art. For example, it is known in the art that quiescent cells, or cells that have stopped dividing, show a decrease in metabolic activity, *i.e.*, glucose uptake, DNA synthesis, RNA synthesis and protein synthesis, required for normal function and growth. Furthermore, simple assays that measure cell division, such as cell counting and incorporation of BrdU into replicating DNA may be used to determine a change in the rate of cell proliferation upon treatment with a dnvMetAP2.

The therapeutic or pharmaceutical compositions of the present invention can be administered by any suitable route known in the art including for example intravenous, subcutaneous, sublingual, intranasal, intramuscular, transdermal, intrathecal, transmucosal, pulmonary inhalation or intracerebral. Administration can be either rapid as by injection or over a period of time as by slow infusion or administration of a slow release formulation as in an, for example, an implantable gel or transdermal patch. For treating tissues in the central nervous system, administration can be by injection or infusion into the cerebrospinal fluid (CSF). When it is intended that an invention dnvMetAP2 be administered to cells in the central nervous system, administration can be with one or more agents capable of promoting penetration of the dnvMetAP2 across the blood-brain barrier.

The invention dnvMetAP2s can also be linked or conjugated with agents that provide desirable pharmaceutical or pharmacodynamic properties. For example, a dnvMetAP2 can be coupled to any substance known in the art to promote penetration or transport across the blood-brain barrier such as an antibody to the transferrin receptor, and administered by intravenous injection (See for example, Friden et al., Science 259:373-377, 1993). Furthermore, a dnvMetAP2 can be stably linked to a polymer such as polyethylene glycol or fused to an albumin moiety to obtain desirable properties of solubility, stability, half-life and other pharmaceutically advantageous properties. (See for example Davis et al. Enzyme Eng 4: 169-73, 1978; Burnham, Am J Hosp Pharm 51: 210-218, 1994).

Preferably, a dnvMetAP2 of the present invention is administered with a carrier such as liposomes or polymers containing a targeting moiety to limit delivery of the dnvMetAP2 to targeted cells. Examples of targeting moieties include but are not limited to antibodies, ligands or receptors to specific cell surface molecules.

For nonparenteral administration, the compositions can also include absorption enhancers which increase the pore size of the mucosal membrane. Such absorption enhancers include sodium deoxycholate, sodium glycocholate, dimethyl-β-cyclodextrin, lauroyl-1-lysophosphatidylcholine and other substances having structural similarities to the phospholipid domains of the mucosal membrane.

The compositions are usually employed in the form of pharmaceutical preparations. Such preparations are made in a manner well known in the pharmaceutical art. One preferred preparation utilizes a vehicle of physiological saline solution, but it is contemplated that other pharmaceutically acceptable carriers such as physiological concentrations of other non-toxic salts, five percent aqueous glucose solution, sterile water or the like may also be used. It may also be desirable that a suitable buffer be present in the composition. Such solutions can, if desired, be lyophilized and stored in a sterile ampoule ready for reconstitution by the addition of sterile water for ready injection. The primary solvent can be aqueous or alternatively non-aqueous. The invention dnvMetAP2s can also be incorporated into a solid or semisolid biologically compatible matrix which can be implanted into tissues requiring treatment.

The carrier can also contain other pharmaceutically-acceptable excipients for modifying or maintaining the pH, osmolarity, viscosity, clarity, color, sterility, stability, rate of dissolution, or odor of the formulation. Similarly, the carrier may contain still other pharmaceutically-acceptable excipients for modifying or maintaining release or absorption or penetration across the blood-brain barrier. Such excipients are those substances usually and customarily employed to formulate dosages for parenteral administration in either unit dosage or multi-dose form or for direct infusion into the cerebrospinal fluid by continuous or periodic infusion.

Dose administration can be repeated depending upon the pharmacokinetic parameters of the dosage formulation and the route of administration used.

It is also contemplated that certain formulations containing a dnvMetAP2 are to be administered orally. Such formulations are preferably encapsulated and formulated with suitable carriers in solid dosage forms. Encapsulation helps to prevent the degradation of the dnvMetAP2 in the digestive tract and may be employed to target the dnvMetAP2 to the appropriate cell type. Encapsulation may, for example, be in the form of proteinoid microsphere carriers, as described in U.S. Pat. No. 4,925,673, which is incorporated herein by reference, or poly amino acid carriers as described in U.S. Pat. No. 6,242,495, which is incorporated herein by reference. Some examples of suitable carriers, excipients, and diluents include lactose, dextrose, sucrose, sorbitol, mannitol, starches, gum acacia, calcium phosphate, alginates, calcium silicate, microcrystalline cellulose, polyvinylpyrrolidone, cellulose, gelatin, syrup, methyl cellulose, methyl- and propylhydroxybenzoates, talc, magnesium, stearate, water, mineral oil, and the like. The formulations can additionally include lubricating agents, wetting agents, emulsifying and suspending agents, preserving agents, sweetening agents or flavoring agents. The compositions may be formulated so as to provide rapid, sustained, or delayed release of the active ingredients after administration to the patient by employing procedures well known in the art. The formulations can also contain substances that diminish proteolytic degradation and promote absorption such as, for example, surface active agents.

The specific dose is calculated according to the approximate body weight or body surface area of the patient or the volume of body space to be occupied. The dose will also be calculated dependent upon the particular route of administration selected. Further refinement of the calculations necessary to determine the appropriate dosage for treatment is routinely made by those of ordinary skill in the art. Such calculations can be made without undue experimentation by one skilled in the art based on the activity of the dnvMetAP2 for a particular cell type *in vitro.* The activity of invention dnvMetAP2s on various cell types in culture is described below and its activity on a particular target cell type can be determined by routine experimentation. Exact dosages are determined in conjunction with standard dose-response studies. It will be understood that the amount of the composition actually administered will be determined by a practitioner, in the light of the relevant circumstances including the condition or conditions to be treated, the choice of composition to be administered, the age, weight, and response of the individual patient, the severity of the patient's symptoms, and the chosen route of administration.

In one embodiment of this invention, an invention dnvMetAP2 may be therapeutically administered by implanting into patients vectors or cells capable of producing a biologically-active form of the dnvMetAP2 or a precursor of the dnvMetAP2, i.e. a molecule that can be readily converted to a biological-active form of the dnvMetAP2 by the body. In one approach cells that secrete the dnvMetAP2 may be encapsulated into semipermeable membranes or polymer matrices for implantation into a patient. It is preferred that the cell be of human origin and that the dnvMetAP2 be derived from human MetAP2 when the patient is human. However, the formulations and methods herein can be used for veterinary as well as human applications and the term "patient" as used herein is intended to include human and veterinary patients.

In another embodiment of this invention, a gene therapy vector comprising any one of the invention polynucleotides that encode a dnvMetAP2 operably linked to a promoter can be administered to a patient. In a preferred embodiment, the gene therapy vector is an adenovirus and the promoter is either a CMV promoter, an endothelial cell-specific promoter or an immune cell-specific promoter. When the patient is a human, the polynucleotide preferably comprises SEQ ID NO:9.

### Embodiments of the Invention: Cell-Based Screen and Assays

Another embodiment of the invention is drawn to a cell-based assay to identify agents that modulate MetAP2 activity. In a preferred embodiment, the assay is a yeast-based synthetic lethal screen, which is well known in the art (Peterson et al., J. Cell Biol. 127:1395-13406, 1994; Bender and Pringle, Mol. Cell. Biol. 11:1295-1305, 1991. Given that the dominant negative effect of a dnvMetAP2 can only be detected in the yeast *Saccharomyces cerevisiae* when the *MAP1* gene is not expressed and no MetAP1 is produced, the synthetic lethal screen may employ a yeast comprising a regulatable *MAP1* gene and a wild-type *MAP2* gene. The yeast cell, which as described herein does not contain an operable naturally occurring chromosomal copy of a gene encoding a MetAP1, is contacted with a prospective agent. If the agent inhibits the activity of MetAP2, either directly or indirectly, then the yeast cell will not grow when *MAP1* is not expressed, and will be able to grow when the *MAP1* gene is expressed. In a preferred embodiment, the yeast strain is *map1*Δ and comprises a *MAP1* gene operably linked to a *GAL1* promoter on a plasmid. The agent may be a polynucleotide, as in a library of genes from any and all organisms, or any molecular compound capable of entering a yeast cell. The yeast cell may be rendered permeable by any method known in the art, such as lithium acetate permeablization or electroporation.

It is further envisioned that a chemical synthetic lethal screen for agents that inhibit MetAP2 may also be conducted in cultured mammalian cells, preferably human cells. An immortalized cell line is engineered to contain a deletion in the naturally occurring chromosomal copy of the gene(s) encoding MetAP1, rendering said naturally occurring chromosomal copy of the gene encoding a MetAP1 inoperable, and an episome that contains a complementing copy of a gene encoding MetAP1 and a gene that encodes a fluorescent protein, such as Green Fluorescent Protein ("GFP") for example. The engineered cell is contacted with a prospective agent. If the agent inhibits MetAP2, the episome comprising the MetAP1 gene, and fortuitously the GFP gene, will be required for viability and thus maintained within the cell. If the agent does not inhibit MetAP2, the episome is not selected for and will therefore be lost from the cell. Given that expression of GFP is lost in the absence of an inhibitor of MetAP2 and maintained in the presence of an inhibitor of MetAP2, a convenient fluorescence-based high throughput assay may be employed to screen for chemical agents that inhibit MetAP2 activity. See Simons et al., Genome Research 11:266-273, 2001, for an enabling description of a human cell based chemical synthetic lethal screen.

Another embodiment of the invention is drawn to a multicopy suppressor-type screen for the identification of downstream effectors of MetAP2. It is envisioned that MetAP2 effects cellular growth and proliferation through downstream effector molecules, such as p53 (*supra*). Therefore, the inventors envision that the overexpression of such a downstream effector of MetAP2 may abrogate the dominant negative effect of dnvMetAP2 on wild-type MetAP2. In a preferred embodiment, a yeast multicopy suppressor screen is employed, wherein the yeast strain is *map1*Δ and comprises a gene encoding a dnvMetAP2, which may be operably linked to a regulatable promoter on a plasmid. Said yeast strain is contacted with a multicopy plasmid comprising a polynucleotide that encodes a downstream effector of MetAP2. In the presence of an overexpressed downstream effector of MetAP2 and in the presence of a dnvMetAP2, the yeast cell may grow. In the absence of a downstream effector and in the presence of a dnvMetAP2, the yeast cell may not grow. The skilled artisan, in the practice of this invention, may employ other yeast strain constructs, such as a *map1*Δ with a complementing *MAP1* gene operably linked to a regulatable promoter, to achieve the same goal of identifying downstream effectors of MetAP2. The inventors envision that said effectors of MetAP2 function may serve as useful reagents and drug targets.

### Embodiments of the Invention: Examples

Preferred embodiments of the invention are described in the following examples. Other embodiments within the scope of the claims herein will be apparent to one skilled in the art from consideration of the specification or practice of the invention as disclosed herein.

The procedures disclosed herein which involve the manipulation of yeast cells, insect cells, mammalian cells and nucleic acids are known to those skilled in the art. *See generally* Fredrick M. Ausubel et al. (1995), "Short Protocols in Molecular Biology", John Wiley and Sons; Joseph Sambrook et al. (1989), "Molecular Cloning, A Laboratory Manual", second ed., ; Celis, J.E., ed., "Cell Biology: A Laboratory Handbook", 2nd edition, Academic Press, San Diego (1998); Dan Burke et al., "Methods in Yeast Genetics: A Cold Spring. Harbor Laboratory Course Manual" (2000 Edition), Cold Spring Harbor Laboratory Press, 2000; Christine Guthrie, "Methods in Yeast Genetics and Molecular Biology", Methods in Enzymology, vol. 194, Academic Press (1990); Harlow and Lane, "Antibodies: A Laboratory Manual", Cold Spring Harbor Laboratory Press, 1988.

### Example 1: Dominant negative yeast MetAP2

### Materials

All materials were from Sigma (St. Louis, MO) unless otherwise stated. Restriction enzymes were from Promega (Madison, WI).

### Bacterial culture and transformation

General handling and techniques for bacteria were followed. Unless otherwise stated, bacteria were cultured in Luria-Bertani (LB) broth (1% bacto-tryptone [Difco], 0.5% yeast extract [Difco] and 1% NaCI). Transformations were carried out using the Z-Competent E. coli Transformation Kit (Zymo Research, Orange, CA) according to manufacturer's protocol. Plasmid DNA was isolated using silica gel-based spin columns and purified using an agarose gel extraction kit (Qiagen).

### Yeast culture and transformation

General handling procedures for yeast were followed (Ausubel et al, "Short Protocols in Molecular Biology", Wiley, which is incorporated herein by reference). Unless otherwise specified, yeast were grown in YPD (1% yeast extract, 2% peptone, and 2% glucose). Synthetic dropout media contained yeast nitrogen base w/o amino acids (Difco), appropriate amino acids to give the desired dropout mixture, and either 2% glucose (for SD medium) or 2% galactose (for SG medium). DNA transformations were performed by the lithium-acetate method (Ito et al, J. Bacteriol. 153:163-168, 1983, which is herein incorporated by reference) using a kit (BIO 101, Vista, CA).

### DNA constructs

*Yeast hemagglutinin-tagged map2 (H174A) mutant:* The wild-type *MAP2* gene containing an N-terminal hemagglutinin (HA) epitope tag (YPYDVPDYA; SEQ ID NO:23) was subcloned from pXL-PE1A (Ghosh et al., J. Bacteriol. 180:4781-4789, 1998) into p425*GPD* (Mumberg et al., Gene 156:119-122, 1995) using the Hind III and Xho I sites. The codon for His¹⁷⁴ was replaced with a codon for Ala in HA*-MAP2* using the QUIKCHANGE^{™} Site-Directed Mutagenesis kit (Stratagene) according to manufacturer's protocol with the following mutagenic primers:

Transformants wee plated on Gibco agar containing isopropyl thio-β-D-galactoside (IPTG; Gold Biotechnologies [St. Louis]) and 5-Bromo-4-chloro-3-indolyl-β-D-galactoside (Xgal; Gold Biotechnologies). Plasmid DNA from positive blue colonies was isolated and sequenced (PE / Applied Biosystems 377). Mutant *map2* (H174A) was then subcloned into p425*GPD* or p425*GAL1* (Mumberg et al, Nuc. Acids Res. 22:5767-5768, 1994) at Hind III / Xho I and sequenced on both strands by an automated sequencer (PE / Applied Biosystems 377).

*Truncated yeast map2* (Δ*2-57* / *H174A):* Residues 2 to 57 of yeast *map2* (H174A) were deleted by PCR-mediated mutagenesis from p425*GPD* / *map2* (H174A) using the following primers:

Amplified, truncated *map2* (Δ2-57 / H174A) was then subcloned into p425*GAL1* at Hind III / Xho I and sequenced.

### Yeast growth assay

For relative growth comparison, strains were grown to mid-logarithmic phase in 5 ml of 2% raffinose minimal media lacking leucine and ∼2 X 10⁵ cells (ABS₆₀₀) were streaked onto SD/Leu⁻ or SG/Leu⁻ plates, then incubated for 4 days at 30°C. For quantitative comparison, a growth curve was obtained (Figure 4). A 50 mL seed culture (SG/Leu⁻) of each strain was grown overnight from SD/Leu⁻ plates until ABS₆₀₀ ∼ 0.1. Each culture was resuspended in 1 mL of SG/Leu to give ABS₆₀₀ ∼ 5.0. The ABS₆₀₀ of each 1 mL culture was measured and diluted to an ABS₆₀₀ of 0.2 in 5 mL of SG/Leu⁻. Each 5 mL culture was then grown aerobically at 30°C and the ABS₆₀₀ was measured in triplicate at one-hour intervals.

### Extraction and purification of HA-tagged yeast MetAP2

The purification of HA-tagged wild-type and mutant yeast MetAP2 from a yeast *map1*Δ strain (Klinkenberg et al., Arch. Biochem. Biophys. 347:193-200, 1997) was performed as previously described (Li and Chang, 1995) with slight modifications. A 1-liter yeast culture was grown aerobically at 30°C in SG/Leu⁻ medium. Cells were collected at an OD₆₀₀ ∼ 1-2 by centrifugation at 1500 x g for 5 min. The pellet was rinsed with buffer XL (10 mM Hepes, pH 7.4; 1.5 mM MgCl₂, 10% Glycerol [v/v]) plus 100 mM NaCl and fresh protease inhibitors (1 µg/ml aprotinin, 1 µg/ml leupeptin, 0.7 µg/ml pepstatin A, 1 mM phenylmethylsulfonyl fluoride). Cells were disrupted by vortexing (5 cycles of 1 min vortexing, 1 min on ice) with 0.5-mm acid-washed glass beads (Biospec Products, Inc.,). The lysate was then cleared at 10,000 x g for 20 min at 4°C. Cleared lysate was applied to a Protein-Sepharose G column (Pharmacia) previously rinsed and pre-equilibrated with buffer XL and an anti-hemagglutinin epitope (YPYDVPDYA; SEQ ID NO:23) monoclonal antibody (BabCO). After loading, the column was washed with buffer XL until OD₂₈₀ < 0.01. The HA-tagged enzyme was then eluted from the immunoaffinity column with 1 mg/ml of free HA polypeptide in buffer XL. After extensive dialysis against buffer XL, each purified protein was assayed for MetAP activity using 2 mM of Met-Gly-Met-Met (SEQ ID NO:24) as described (Li and Chang, 1995).

### Generation of polyclonal antibodies against yeast MetAP2

A standard procedure was adapted (Harlow and Lane, *supra*). An oligopeptide corresponding to a conserved 10-amino acid sequence at the C-terminus of MetAP2 (CKEWSKGDDY) (SEQ ID NO:22) was obtained (Research Genetics). An N-terminal cysteine was included in the peptide for coupling to the carrier protein, maleimide-activated keyhole limpet hemocyanin (KLH). The peptide and KLH were conjugated at 2 mg peptide/ 2 mg KLH according to the manufacturer's protocol (Pierce). The KLH-peptide immunogen was mixed with an equal volume of Freund's complete adjuvant (Difco) and 400 µl of this emulsion was injected intramuscularly into each thigh of a host rabbit. Two booster injections of the same amount of antigen emulsified in incomplete Freund's adjuvant (Sigma) were given at weeks 4 and 8. Blood was collected from the ear prior to the initial injection and 10 days after each boost. Blood samples stood at room temperature for 4 hours before being placed at 4°C overnight. Coagulated blood was cleared by centrifugation at 3000 x g for 10 min. The MetAP2 antibodies were purified using cyanogen bromide-activated thiol-sepharose (Sigma) coupled with the peptide antigen. Antisera specificity and titer from each collection was examined by immunoblot against immunoaffinity-purified epitope-tagged yeast MetAP2 (Li and Chang, 1995)

### Polyacrylamide gel electrophoresis and Western blots

SDS-PAGE (Laemmli, Nature 227:680-685, 1970) was performed on 10% polyacrylamide gels. The total protein concentration of crude extracts was determined by the Bradford assay (Bradford, Anal. Biochem. 72:248-254, 1976). Gels were wet-transferred overnight to a nitrocellulose membrane and blocked for 1 hour with Tris-buffered saline solution containing 0.2% Tween 20 (TBST) plus 5% nonfat dry milk. All western blots were performed following the ECL^{™} detection protocol (Amersham). Membranes were incubated with rabbit anti-yeast MetAP2 polyclonal antibodies (1:500) in TBST plus 1% nonfat dry milk for 1 hour at room temperature. Membranes were then incubated with goat anti-rabbit horseradish peroxidase conjugated antibodies (1:6000, Sigma) for 30 minutes at room temperature and exposed to x-ray film (Molecular Technologies, St. Louis, MO).

### Results

*MetAP2 (H174A) is a dysfunctional catalyst -* To generate a catalytically inactive MetAP2 mutant, the codon for His¹⁷⁴ of the wild-type yeast *MAP2* gene containing an N-terminal hemagglutinin epitope tag (Li and Chang, 1995) was replaced with the codon for alanine by site-directed mutagenesis. The resultant mutant gene, *map*2 (H174A), was subcloned into a multi-copy vector under the strong yeast glyceraldehyde 3-phosphate (*GPD*) promoter (p425*GPD*) (Mumberg, 1995).

In order to minimize background aminopeptidase activity with purified MetAP2 (H174A), a yeast *map1*Δ strain (Klinkenberg, 1997) was transformed with p425*GPD* / *map2* (H174A). No colonies were obtained when *map1*Δ was transformed with p425*GPD* / *map2* (H174A) which initially suggested that MetAP2 (H174A) overexpression is lethal in *map1*Δ. Overexpression of MetAP2 (H174A) in a multi-copy vector under the relatively weaker, regulatable *GAL1* promoter (Mumberg, 1994), however, was not lethal and allowed for the purification of MetAP2 (H174A).

Wild-type MetAP2 and MetAP2 (H174A), each having an N-terminal hemagglutinin (HA)-tag (YPYDVPDYA) (SEQ ID NO:23), were immunopurified from *map1*Δ with mouse anti-HA epitope monoclonal antibodies and the catalytic activity of each enzyme was assessed. Unlike wild-type MetAP2, MetAP2 (H174A) displayed no detectable activity against a peptide substrate (MGMM) (SEQ ID NO:24) *in vitro* (Fig. 2). Thus, replacement of conserved His¹⁷⁴ with alanine disrupts the catalytic activity of yeast MetAP2.

*Overexpression of map2 (H174A) in map1Δ under the GPD promoter is lethal-*Removal of N-terminal methionine is essential for cellular growth in yeast (Chang et al., 1992; Li and Chang, 1995). Thus, if overexpression of a mutant MetAP2 in *map1*Δ interferes with wild-type MetAP2 function, a slow-growth or lethal phenotype is expected. To test whether MetAP2 (H174A) interferes with wild-type MetAP2 function, a yeast *map1*Δ strain, YHC001 (Klinkenberg, 1997) (expresses only wild-type MetAP2), was transformed with p425*GPD* / *map2* (H174A). The steady state levels of MetAP2 in the *map1*Δ strain and the originating wild-type yeast strain (W303-1A) are similar (data not shown). No colonies were obtained after two consecutive transformations of *map1*Δ with -0.1 µg of p425*GPD* / *map2* (H714A) while -170 colonies were obtained with -0.1 µg of vector alone (data not shown). This finding suggested that overexpression of MetAP2 (H174A) in *map1*Δ under the *GPD* promoter is lethal.

*GAL1 expression of map2 (H174A) inhibits the growth of map1*Δ *-* A qualitative growth comparison was performed to assess whether expression of MetAP2 (H174A) under *GAL1* inhibits the growth of *map1*Δ. Colonies of equal size were observed on glucose plates where expression under *GAL1* is repressed (Fig. 3A). In contrast, colonies of different sizes were observed on galactose plates where expression is activated (Fig. 3B).

Colonies of *map1*Δ overexpressing wild-type MetAP2 were the largest observed (Fig. 3B, WT). This finding is consistent with our previous observation that overexpression of wild-type MetAP2 can almost completely complement the slow-growth phenotype of the *map1*Δ strain. Colonies of *map1*Δ transformed with MetAP2 (H174A) were observed on both glucose and galactose plates (Fig. 3, H174A). Colonies of *map1*Δ with vector only, however, were larger than colonies of *map1*Δ overexpressing MetAP2 (H174A) on galactose (Fig. 3B, H174A & vector). A growth rate assay confirmed that the doubling time of *map1*Δ overexpressing MetAP2 (H174A) under GAL1 (9.3 ± 0.6 hrs) is greater than the doubling time of *map1*Δ alone (6.0 ± 0.5 hrs) (Figure 4). Thus, these findings indicate that overexpression of H174A-MetAP1 under *GAL1* inhibits the growth rate of *map1*Δ.

*Yeast MetAP2 H174A requires its N-terminal domain for maintenance of the dominant negative effect -* We previously proposed that the polylysine block of yeast MetAP2 mediates the presumed ribosome association of MetAP2 (Li and Chang, 1995). Thus, if ribosome association is required for MetAP2 (H174A) to interfere with wild-type MetAP2 function, it is expected that removal of the polylysine block will preclude the MetAP2 (H174A) dominant negative phenotype in *map1*Δ*.*

Codons for residues 2-57, which comprises the N-terminal polylysine block, were removed from *map2* (H174A) by PCR-mediated mutagenesis. The truncated mutant MetAP2 (Δ2-57 / H174A) was then expressed in *map1*Δ under the *GAL1* promoter to assess its effect on *map1*Δ growth.

*map1*Δ strains expressing truncated MetAP2 (Δ2-57 / H174A) grew at the same rate and generated colonies that were the same size as *map1*Δ with vector alone (Figure 4; Fig. 5B, V & Δ2-57). This is in contrast to the smaller colonies of *map1*Δ expressing full length MetAP2 (H174A) (Fig. 5B, H174A). The doubling time of MetAP2 (Δ2-57 / H174A) (7.0 ± 0.6 hrs) was also similar to *map1*Δ with vector only (6.0 ± 0.5 hrs) (Figure 4). Thus, these data indicate that MetAP2 (H174A) requires its N-terminal domain for maintenance of the dominant negative effect in *map1*Δ.

*The steady state levels of each MetAP2 construct are similar -* The steady state level of each MetAP2 construct was determined. Wild-type, H174A, and truncated H174A were expressed at similar levels in *map1*Δ under the *GAL1* promoter (Fig. 6). A smaller fragment was observed with MetAP2 (H174A) that was not observed with wild-type MetAP2 (Fig. 6, lanes 1 & 3). A similar, less obvious fragment of approximately the same size is observed when wild-type MetAP2 is expressed in *map2*Δ under the *GPD* promoter (unpublished observation).

*Overexpression of MetAP2 (H174A) has little effect on map2*Δ *growth -* We have reported evidence that a dominant negative mutant of yeast MetAP1 (D219N) interferes with wild-type MetAP2 function (Klinkenberg, 1997). To determine if MetAP2 (H174A) interferes with the function of wild-type MetAP1 in a reciprocal manner, MetAP2 (H174A) was overexpressed under the strong *GPD* promoter in a *map2*Δ strain (Klinkenberg, 1997) (only MetAP1 is expressed).

Colonies of *map2*Δ overexpressing MetAP2 (H174A) were similar in size to *map2*Δ with vector alone (Fig. 7, H174A & V). Colonies of *map2*Δ overexpressing wild-type MetAP2 were also similar to *map2*Δ with vector alone (Fig. 7, WT & V). These results indicate that overexpression of wild-type or MetAP2 (H174A) in *map2*Δ under the *GPD* promoter does not affect the growth of *map2*Δ*.* Thus, these findings suggest that MetAP2 (H174A) does not interfere with wild-type MetAP1 function.

### Example 2: Production of human Dominant Negative MetAP2

### Generation of recombinant virus.

The open reading frame of the human *MAP2* cDNA, which encodes human MetAP2, was subcloned into the pcDNA3.1 vector in a sense or anti-sense orientation, thus placing the gene under the control of a CMV promoter (Figure 8). The recombinant transfer vector was then constructed as follows:
The following two oligonucleotide primers were used to amplify a DNA fragment from pcDNA3.1-hMAP2.:
5' -CAC AGA ATT CCC CGC ATC CCC AGC ATG CCT GCT ATT G- 3' (SEQ ID NO:26)

The resultant fragment included the CMV promoter, hMAP2 open reading frame (sense), and polyA signal sequence. Two unique restriction sites, Xhol and Hind III, were introduced at the 5'-end and 3'-end of the PCR product, respectively. This PCR product was then inserted into the corresponding sites in the pQBI-AdBN vector (Figure 9).

### Generation of recombinant virus -

Sub-confluent QBI-293A cells were co-transfected with 5 µg of QBI-viral DNA and 5 µg of linearized transfer plasmid containing the sense h*MAP2* cDNA in a 60 mm petri dish using the standard calcium phosphate technique. - 50 plaques, with > 45% being recombinant virus were obtained. As a positive control, 0.5 µg of QBI-Transfer⁺ with 10 µg of QBI-carrier DNA was also used for transfection. About 150 plaques were obtained.

### Screening, purification, amplification, and titering of adenovirus recombinants:

Recombinant plaques were identified by PCR using the same primers described above. In short, 20 plaques were analyzed for each construct. PCR was performed with 30 cycles of amplification using Taq polymerase and about 4 µl of viral stock without any extraction procedure. After the first screening, five (5) plaques were purified and three (3) clones were selected that produced the strongest PCR signal for further analysis.

Next, the recombinant adenovirus was amplified, and the titer of each clone was determined as described in the manufacturer's protocols (QBI, Canada). Viral stocks were stored for the following experiments. The recombinant virus containing the *hMAP2* cDNA is designated as Ad*hMAP2*.

### Generation of recombinant mutant virus:

The recombinant transfer vector containing mutated hMAP2 cDNA was constructed as follows. The recombinant transfer vector AdBN-*hMAP2* (HA-tagged) was used and HiS²³¹ was mutated to Ala by site-directed mutagenesis. This linearized recombinant transfer vector was then used to cotransfect the QBI-293 cells with QBI-viral DNA by the same procedures as described above. The recombinant virus was screened, purified, characterized, and used to examine the correlation between the expression level of the dominant negative MetAP2 mutant and cell growth.

### Example 3: Effect of dnvMetAP2 on Human Vascular Endothelial Cells

The expression level of the HA-tagged MetAP2 (H231A) was tested in human umbilical vascular endothelial ("HUVE") cells infected with Ad*MAP2* (H231A) at a multiplicity of infection ("MOI") = 0.2 -20.0. At 2 days after viral infection, 50 µL of culture was harvested. One part of the harvested culture was used for cell proliferation assays according to manufacturer's procedures, which are incorporated herein by reference (Cell Counting kit-8, Dojindo Molecular Technologies, Inc, MD). According to Table 1 and Figure 10, the rate of proliferation of HUVE cells that were transfected with an adenovirus vector comprising a human dnvMetAP2 gene [AdhMAP2(H231A)] was reduced 66% of the rate of control HUVE cells transfected with either an empty adenovirus vector [AdBN(-hMAP2)] or an adenovirus vector comprising the wild-type human MetAP2 gene (AdhMAP2). These results unequivocally demonstrate that dnvMetAP2 blocks human cell proliferation, especially endothelial cell proliferation, which is a necessary step in angiogenesis. These results further demonstrate the effectiveness of the adenoviral gene delivery system in administering dnvMetAP2 to vascular endothelial cells.

**Table 1: Relative proliferation rate (%) of HUVECS transfected with increasing amounts of adenoviral vectors compared to non-transfected HUVECS.**

| M.O.I. | AdBN (-hMAP2) | AdhMAP2 | AdhMAP2 (H231A) |
|---|---|---|---|
| 0.2 | 100 | 100 | 100 |
| 0.4 | 100 | 100 | 99 |
| 0.6 | 100 | 99 | 95 |
| 2.0 | 100 | 99 | 89 |
| 4.0 | 99 | 99 | 34 |

To demonstrate expression of the MetAP2 and dnvMetAP2 proteins, another part of the harvested culture was analyzed by SDS/PAGE, transferred to nitrocellulose membrane, and then blotted with anti-HA monoclonal antibodies followed by incubation with secondary anti-mouse IgG-horseradish peroxidase conjugate. The signal is detected by enhanced chemiluminescence, according to manufacturer's instructions (Amersham). Endothelial cells infected with Ad*hMAP2*(wt) virus at the same MOI value are used as a control. Figure 11A depicts the developed western blot, showing expression of both wild-tyoe human MetAP2 (lane b) and human dnvMetAP2 (lane c).

The discussion of references herein is intended merely to summarize the assertions made by their authors and no admission is made that any reference constitutes prior art. Applicants reserve the right to challenge the accuracy and pertinence of the cited references.

## Claims

1. An *in vitro* method for inhibiting cell growth comprising contacting the cell with:
(i) a composition comprising an isolated and purified polypeptide, wherein the polypeptide is a variant MetAP2 that has dominant negative MetAP2 activity and contains a translation domain or;
(ii) a composition comprising an isolated and purified polynucleotide, wherein the polynucleotide encodes a variant MetAP2 that has dominant negative MetAP2 activity and contains a translation domain.

2. The method of claim 1 wherein the polypeptide comprises a sequence that is at least 46% identical to SEQ ID NO: 6, SEQ ID NO: 7, SEQ ID NO: 8 or SEQ ID NO: 16.

3. The method of claim 1 wherein the polypeptide comprises SEQ ID NO: 6, SEQ ID NO: 7, SEQ ID NO: 8 or SEQ ID NO: 16.

4. The method of claim 1 wherein the polypeptide consists of SEQ ID NO: 6, SEQ ID NO: 7, SEQ ID NO: 8 or SEQ ID NO: 16.

5. The method of claim 1 wherein the polypeptide consists of SEQ ID NO: 12, wherein the histidine at position number 231 is replaced with an alanine.

6. The method of claim 1 wherein the polynucleotide comprises a sequence selected from SEQ ID NO: 9, SEQ ID NO: 10, SEQ ID NO: 11 and SEQ ID NO: 18.

7. The method of claim 6 wherein the polynucleotide comprises SEQ ID NO: 9.

8. The method of claim 1 wherein the cell is from a subject suffering from a disease mediated by a fungal infection, cell proliferation, angiogenesis, decreased function of p53 or immune system activity,

9. The method of claim 8 wherein the subject is a human suffering from a disease mediated by angiogenesis.

10. The method of claim 1 wherein the polynucleotide is part of an adenovirus vector and operably linked to a CMV promoter.

11. The use of a composition comprising an isolated and purified polypeptide which is, or an isolated and purified polynucleotide encoding, a variant MetAP2 that has dominant negative MetAP2 activity and contains a translation domain in the manufacture of a medicament for the treatment of a disease mediated by a fungal infection, cell proliferation, angiogenesis, decreased function of p53 or immune system activity.

12. An *in vitro* method of identifying an agent that modulates the activity of MetAP2 comprising contacting a cell with an agent, wherein:
(a) the cell contains a functional gene that encodes a MetAP2 and does not contain an operable naturally occurring chromosomal copy of a gene encoding MetAP1; and
(b) the modulation activity of the agent is determined by measuring either the relative growth rate of the cell or the fluorescence emission of the cell;
wherein either:
A.
(a) the cell is a yeast cell which comprises a gene encoding MetAP1 operably linked to a regulatable promoter, and
(b) the modulation activity of the agent is determined by comparing the growth rate of the yeast cell in the absence of MetAP1 expression to the growth rate of the yeast cell in the presence of MetAP1 expression; or
B.
(a) the cell is a mammalian cell which comprises a polynucleotide that further comprises a gene encoding a MetAP1 and a gene encoding a fluorescent protein, and
(b) the modulation activity of the agent is determined by measuring the fluorescence emission of the cell.; optionally wherein the agent is a polynucleotide.

13. A method of identifying effectors of MetAP2 activity comprising contacting a yeast cell with a polynucleotide and determining that the polynucleotide encodes an effector of MetAP2 activity, wherein:
(a) the yeast cell comprises a functional gene that encodes a MetAP2 and a polynucleotide that encodes a dominant negative MetAP2;
(b) the yeast cell does not contain an operable naturally occurring chromosomal copy of a gene encoding a MetAP1; and
(c) the determining step comprises comparing the growth rate of yeast cells that contain a polynucleotide that encodes an effector of MetAP2 activity with a yeast cell that does not contain a polynucleotide that encodes an effector of MetAP2 activity, wherein the growth rate of a yeast cell that contains a polynucleotide that encodes an effector of MetAP2 activity is greater than the growth rate of a yeast cell that does not contain a polynucleotide that encodes an effector of MetAP2 activity,

14. The method of claim 13 wherein the polynucleotide is a human polynucleotide.

## Patentansprüche

1. In-vitro-Verfahren zur Hemmung der Zellvermehrung, umfassend das Kontaktieren der Zelle mit:
(i) einer Zusammensetzung, umfassend ein isoliertes und gereinigtes Polypeptid, worin das Polypeptid eine MetAP2-Variante ist, die dominante negative MetAP2-Aktivität aufweist und eine Translationsdomäne enthält, oder
(ii) einer Zusammensetzung, umfassend ein isoliertes und gereinigtes Polynucleotid, worin das Polynucleotid für eine MetAP2-Variante kodiert, die dominante negative MetAP2-Aktivität aufweist und eine Translationsdomäne enthält.

2. Verfahren nach Anspruch 1, worin das Polypeptid eine Sequenz umfasst, die zu mindestens 46 % identisch mit Seq.-ID Nr. 6, Seq.-ID Nr. 7, Seq.-ID Nr. 8 oder Seq.-ID Nr. 16 ist.

3. Verfahren nach Anspruch 1, worin das Polypeptid Seq.-ID Nr. 6, Seq.-ID Nr. 7, Seq.-ID Nr. 8 oder Seq.-ID Nr. 16 umfasst.

4. Verfahren nach Anspruch 1, worin das Polypeptid aus Seq.-ID Nr. 6, Seq.-ID Nr. 7, Seq.-ID Nr. 8 oder Seq.-ID Nr. 16 besteht.

5. Verfahren nach Anspruch 1, worin das Polypeptid aus Seq.-ID Nr. 12 besteht, worin das Histidin auf Position 231 durch ein Alanin ersetzt ist.

6. Verfahren nach Anspruch 1, worin das Polynucleotid eine aus Seq.-ID Nr. 9, Seq.-ID Nr. 10, Seq.-ID Nr. 11 und Seq.-ID Nr. 18 ausgewählte Sequenz umfasst.

7. Verfahren nach Anspruch 6, worin das Polynucleotid Seq.-ID Nr. 9 umfasst.

8. Verfahren nach Anspruch 1, worin die Zelle aus einem Individuum stammt, das an einer durch eine Pilzinfektion, Zellproliferation, Angiogenese, verminderte Funktion von p53 oder Immunsystemaktivität vermittelten Krankheit leidet.

9. Verfahren nach Anspruch 8, worin das Individuum ein Mensch ist, der an einer durch Angiogenese vermittelten Krankheit leidet.

10. Verfahren nach Anspruch 1, worin das Polynucleotid Teil eines Adenovirusvektors und operabel mit einem CMV-Promotor verbunden ist.

11. Verwendung einer Zusammensetzung, umfassend ein isoliertes und gereinigtes Polypeptid, welches eine MetAP2-Variante ist, oder ein isoliertes und gereinigtes Polynucleotid, welches für eine MetAP2-Variante kodiert, die dominante negative MetAP2-Aktivität aufweist und eine Translationsdomäne enthält, zur Herstellung eines Medikaments zur Behandlung einer durch eine Pilzinfektion, Zellproliferation, Angiogenese, verminderte Funktion von p53 oder Immunsystemaktivität vermittelten Krankheit.

12. In-vitro-Verfahren zur Identifikation eines Mittels, das die Aktivität von MetAP2 moduliert, umfassend das Kontaktieren einer Zelle mit einem Mittel, worin:
(a) die Zelle ein funktionelles Gen enthält, das für eine MetAP2 kodiert und keine operable, natürlich vorkommende chromosomale Kopie eines für MetAP1 kodierenden Gens enthält; und
(b) die Modulierungsaktivität des Mittels durch Messung entweder der relativen Vermehrungsgeschwindigkeit der Zelle oder der Fluoreszenzemission der Zelle bestimmt wird;
worin entweder:
A.
(a) die Zelle eine Hefezelle ist, die ein Gen umfasst, welches für MetAP1 kodiert und an einen regulierbaren Promotor operabel gebunden ist, und
(b) die Modulierungsaktivität des Mittels durch Vergleich der Vermehrungsgeschwindigkeit der Hefezelle in Abwesenheit von MetAP1-Expression mit der Vermehrungsgeschwindigkeit der Hefezelle in Gegenwart von MetAP1-Expression bestimmt wird; oder
B.
(a) die Zelle eine Säugetierzelle ist, die ein Polynucleotid, welches weiters ein Gen, das für eine MetAP1 kodiert, und ein Gen, das für ein fluoreszierendes Protein kodiert, umfasst, und
(b) die Modulierungsaktivität des Mittels durch Messung der Fluoreszenzemission der Zelle bestimmt wird;
worin gegebenenfalls das Mittel ein Polynucleotid ist.

13. Verfahren zur Identifikation von Effektoren der MetAP2-Aktivität, umfassend das Kontaktieren einer Hefezelle mit einem Polynucleotid und das Bestimmen, dass das Polynucleotid für einen Effektor der MetAP2-Aktivität kodiert, worin:
(a) die Hefezelle ein funktionelles Gen, das für eine MetAP2 kodiert, und ein Polynucleotid, das für eine dominante negative MetAP2 kodiert, umfasst;
(b) die Hefezelle keine operable, natürlich vorkommende chromosomale Kopie eines Gens enthält, das für eine MetAP1 kodiert; und
(c) der Bestimmungsschritt den Vergleich der Vermehrungsgeschwindigkeit der Hefezellen, die ein für einen Effektor der MetAP2-Aktivität kodierendes Polynucleotid enthalten, mit einer Hefezelle, die kein für einen Effektor der MetAP2-Aktivität kodierendes Polynucleotid enthält, umfasst, worin die Vermehrungsgeschwindigkeit einer Hefezelle, die ein für einen Effektor der MetAP2-Aktivität kodierendes Polynucleotid enthält, höher ist als die Vermehrungsgeschwindigkeit einer Hefezelle, die kein für einen Effektor der MetAP2-Aktivität kodierendes Polynucleotid enthält.

14. Verfahren nach Anspruch 13, worin das Polynucleotid ein menschliches Polynucleotid ist.

## Revendications

1. Méthode in vitro pour inhiber la croissance de cellule comprenant la mise en contact de la cellule avec:
(i) une composition comprenant un polypeptide isolé et purifié, où le polypeptide est une variante MetAP2 qui a une activité négative dominante de MetAP2 et contient un domaine de translation ou
(ii) une composition comprenant un polynucléotide isolé et purifié, où le polynucléotide code pour une MetAP2 variante qui a une activité dominante négative de MetAP2 et contient un domaine de translation.

2. Méthode selon la revendication 1, dans laquelle le polypeptide comprend une séquence qui est au moins à 46% identique à SEQ ID NO:6, SEQ ID NO:7, SEQ ID NO: 8 ou SEQ ID NO:16.

3. Méthode selon la revendication 1, dans laquelle le polypeptide comprend SEQ ID NO:6, SEQ ID NO:7, SEQ ID NO:8 ou SEQ ID NO:16.

4. Méthode selon la revendication 1, dans laquelle le polypeptide consiste en SEQ ID NO:6, SEQ ID NO:7, SEQ ID NO:8 ou SEQ ID NO:16.

5. Méthode selon la revendication 1, dans laquelle le polypeptide consiste en SEQ ID NO:12, où l'histidine au numéro de position 231 est remplacée par une alanine.

6. Méthode selon la revendication 1, où le polynucléotide comprend une séquence sélectionnée parmi SEQ ID NO:9, SEQ ID NO:10, SEQ ID NO:11 et SEQ ID NO:18.

7. Méthode selon la revendication 6, où le polynucléotide comprend SEQ ID NO:9.

8. Méthode selon la revendication 1, dans laquelle la cellule provient d'un sujet souffrant d'une maladie provoquée par une infection fongique, prolifération de cellules, angiogénèse, fonction diminuée de p53 ou de l'activité du système immunitaire.

9. Méthode selon la revendication 8, où le sujet est un être humain souffrant d'une maladie provoquée par l'angiogénèse.

10. Méthode selon la revendication 1, où le polynucléotide fait partie d'un vecteur adénoviral et est lié fonctionnellement à un promoteur de CMV.

11. Utilisation d'une composition comprenant un polypeptide isolé et purifié qui est, ou un polynucléotide isolé et purifié codant pour, une variante de MetAP2 qui a une activité dominante négative de MetAP2 et contient un domaine de translation dans la fabrication d'un médicament pour le traitement d'une maladie provoquée par une infection fongique, prolifération de cellules, angiogénèse, fonction diminuée de p53 ou de l'activité du système immunitaire.

12. Méthode in vitro pour identifier un agent qui module l'activité de la MetAP2 comprenant la mise en contact d'une cellule avec un agent, dans laquelle:
(a) la cellule contient un gêne fonctionnel qui code pour une MetAP2 et ne contient pas de copie chromosomique utilisable se produisant naturellement d'un gêne codant pour la MetAP1; et
(b) l'activité de modulation de l'agent est déterminée en mesurant soit la vitesse de croissance relative de la cellule soit l'émission de fluorescence de la cellule;
dans laquelle soit:
A.
(a) la cellule est une cellule de levure qui comprend un gêne codant pour la MetAP1 fonctionnellement lié à un promoteur réglable, et
(b) l'activité de modulation de l'agent est déterminée en comparant la vitesse de croissance de la cellule de levure en l'absence de l'expression de MetAP1 avec la vitesse de croissance de la cellule de levure en présence de l'expression de la MetAP1; ou
B.
(a) la cellule est une cellule de mammifère qui comprend un polynucléotide qui comprend en outre un gêne codant pour la MetAP1 et un gêne codant pour une protéine fluorescente; et
(b) l'activité de modulation de l'agent est déterminée en mesurant l'émission de fluorescence de la cellule;
optionnellement, où l'agent est un polynucléotide.

13. Méthode d'identification d'effecteurs de l'activité de la MetAP2 comprenant la mise en contact d'une cellule de levure avec un polynucléotide et la détermination que le polynucléotide code pour un effecteur de l'activité de MetAP2, où
(a) la cellule de levure comprend un gêne fonctionnel qui code pour une MetAP2 et un polynucléotide qui code pour une MetAP2 dominante négative;
(b) la cellule de levure ne contient pas de copie chromosomique utilisable se produisant naturellement d'un gêne codant pour une MetAP1; et
(c) l'étape de détermination comprend la comparaison de la vitesse de croissance des cellules de levure qui contiennent un polynucléotide qui code pour un effecteur de l'activité de MetAP2 avec une cellule de levure qui ne contient pas de polynucléotide qui code pour un effecteur de l'activité de MetAP2, où la vitesse de croissance d'une cellule de levure qui contient un polynucléotide qui code pour un effecteur de l'activité de MetAP2 est plus grande que la vitesse de croissance d'une cellule de levure qui ne contient pas de polynucléotide qui code pour un effecteur de l'activité de MetAP2.

14. Méthode selon la revendication 13, où le polynucléotide est un polynucléotide humain.


### SEQUENCE LISTING

<110> CHANG, Y-H
   VETRO, J.A.
   MICKA, W.S.
<120> Dominant Negative Variants of Methionine Aminopeptidase 2 ("MetAP2") and Clinical Uses Therefor
<130> 16153-8007
<140>
   <141>
<160> 26
<170> PatentIn Ver. 2.0
<210> 1
   <211> 71
   <212> PRT
   <213> Human polylysine
<400> 1
   Lys Lys Lys Arg Arg Lys Lys Lys Lys Ser Lys Gly Pro Ser Ala Ala
   1               5                   10                  15
   Gly Glu Gln Glu Pro Asp Lys Glu Ser Gly Ala Ser Val Asp Glu Val
               20                  25                  30
   Ala Arg Gln Leu Glu Arg Ser Ala Leu Glu Asp Lys Glu Arg Asp Glu
           35                  40                  45
   Asp Asp Glu Asp Gly Asp Gly Asp Gly Asp Gly Ala Thr Gly Lys Lys
       50                  55                  60
   Lys Lys Lys Lys Lys Lys Lys
   65                  70
<210> 2
   <211> 71
   <212> PRT
   <213> Mouse polylysine
<400> 2
   Lys Lys Lys Arg Arg Lys Lys Lys Lys Gly Lys Gly Ala Val Ser Ala
   1               5                   10                  15
   Val Gln Gln Glu Leu Asp Lys Glu Ser Gly Ala Leu Val Asp Glu Val
               20                  25                  30
   Ala Lys Gln Leu Glu Ser Gln Ala Leu Glu Glu Lys Glu Arg Asp Asp
           35                  40                  45
   Asp Asp Glu Asp Gly Asp Gly Asp Ala Asp Gly Ala Thr Gly Lys Lys
       50                  55                  60
   Lys Lys Lys Lys Lys Lys Lys
   65                  70
<210> 3
   <211> 57
   <212> PRT
   <213> Saccharomyces polylysine
<400> 3
   Thr Asp Ala Glu Ile Glu Asn Ser Pro Ala Ser Asp Leu Lys Glu Leu
   1               5                   10                  15
   Asn Leu Glu Asn Glu Gly Val Glu Gln Gln Asp Gln Ala Lys Ala Asp
               20                  25                  30
   Glu Ser Asp Pro Val Glu Ser Lys Lys Lys Lys Asn Lys Lys Lys Lys
           35                  40                  45
   Lys Lys Lys Ser Asn Val Lys Lys Ile
       50                  55
<210> 4
   <211> 35
   <212> DNA
   <213> Synthetic oligonucleotide
<400> 4
   caaccattgt gctgcagctt tcacacccaa tgcag 35
<210> 5
   <211> 35
   <212> DNA
   <213> Artificial Sequence
<220>
   <223> Description of Artificial Sequence: synthetic oligonucleotide
<400> 5
   ctgcattggg tgtgaaagct gcagcacaat ggttg 35
<210> 6
   <211> 478
   <212> PRT
   <213> Human dnvMetAP2
<220>
   <221> SITE
   <222> (219)
   <223> May be any naturally occurring amino acid
<220>
   <221> SITE
   <222> (231)
   <223> May be any amino acid, except His
<220>
   <221> SITE
   <222> (251)
   <223> May be any naturally occurring amino acid
<220>
   <221> SITE
   <222> (262)
   <223> May be any naturally occurring amino acid
<220>
   <221> SITE
   <222> (328)
   <223> May be any naturally occurring amino acid
<220>
   <221> SITE
   <222> (331)
   <223> May be any naturally occurring amino acid
<220>
   <221> SITE
   <222> (338)..(339)
   <223> May be any naturally occurring amino acid
<220>
   <221> SITE
   <222> (364)
   <223> May be any naturally occurring amino acid
<220>
   <221> SITE
   <222> (444)
   <223> May be any naturally occurring amino acid
<220>
   <221> SITE
   <222> (447)
   <223> May be any naturally occurring amino acid
<220>
   <221> SITE
   <222> (459)
   <223> May be any naturally occurring amino acid
<400> 6
   Met Ala Gly Val Glu Glu Val Ala Ala Ser Gly Ser His Leu Asn Gly
    1               5                  10                  15
   Asp Leu Asp Pro Asp Asp Arg Glu Glu Gly Ala Ala Ser Thr Ala Glu
               20                  25                  30
   Glu Ala Ala Lys Lys Lys Arg Arg Lys Lys Lys Lys Ser Lys Gly Pro
           35                  40                  45
   Ser Ala Ala Gly Glu Gln Glu Pro Asp Lys Glu Ser Gly Ala Ser Val
       50                  55                  60
   Asp Glu Val Ala Arg Gln Leu Glu Arg Ser Ala Leu Glu Asp Lys Glu
   65                  70                  75                  80
   Arg Asp Glu Asp Asp Glu Asp Gly Asp Gly Asp Gly Asp Gly Ala Thr
                   85                  90                  95
   Gly Lys Lys Lys Lys Lys Lys Lys Lys Lys Arg Gly Pro Lys Val Gln
               100                 105                 110
   Thr Asp Pro Pro Ser Val Pro Ile Cys Asp Leu Tyr Pro Asn Gly Val
           115                 120                 125
   Phe Pro Lys Gly Gln Glu Cys Glu Tyr Pro Pro Thr Gln Asp Gly Arg
       130                 135                 140
   Thr Ala Ala Trp Arg Thr Thr Ser Glu Glu Lys Lys Ala Leu Asp Gln
   145                 150                 155                 160
   Ala Ser Glu Glu Ile Trp Asn Asp Phe Arg Glu Ala Ala Glu Ala His
                   165                 170                 175
   Arg Gln Val Arg Lys Tyr Val Met Ser Trp Ile Lys Pro Gly Met Thr
               180                 185                 190
   Met Ile Glu Ile Cys Glu Lys Leu Glu Asp Cys Ser Arg Lys Leu Ile
   195 200 205
   Lys Glu Asn Gly Leu Asn Ala Gly Leu Ala Xaa Pro Thr Gly Cys Ser
   210 215 220
   Leu Asn Asn Cys Ala Ala Xaa Tyr Thr Pro Asn Ala Gly Asp Thr Thr
   225 230 235 240
   Val Leu Gln Tyr Asp Asp Ile Cys Lys Ile Xaa Phe Gly Thr His Ile
   245 250 255
   Ser Gly Arg Ile Ile Xaa Cys Ala Phe Thr Val Thr Phe Asn Pro Lys
   260 265 270
   Tyr Asp Thr Leu Leu Lys Ala Val Lys Asp Ala Thr Asn Thr Gly Ile
   275 280 285
   Lys Cys Ala Gly Ile Asp Val Arg Leu Cys Asp Val Gly Glu Ala Ile
   290 295 300
   Gln Glu Val Met Glu Ser Tyr Glu Val Glu Ile Asp Gly Lys Thr Tyr
   305 310 315 320
   Gln Val Lys Pro Ile Arg Asn Xaa Asn Gly Xaa Ser Ile Gly Gln Tyr
   325 330 335
   Arg Xaa Xaa Ala Gly Lys Thr Val Pro Ile Val Lys Gly Gly Glu Ala
   340 345 350
   Thr Arg Met Glu Glu Gly Glu Val Tyr Ala Ile Xaa Thr Phe Gly Ser
   355 360 365
   Thr Gly Lys Gly Val Val His Asp Asp Met Glu Cys Ser His Tyr Met
   370 375 380
   Lys Asn Phe Asp Val Gly His Val Pro Ile Arg Leu Pro Arg Thr Lys
   385 390 395 400
   His Leu Leu Asn Val Ile Asn Glu Asn Phe Gly Thr Leu Ala Phe Cys
   405 410 415
   Arg Arg Trp Leu Asp Arg Leu Gly Glu Ser Lys Tyr Leu Met Ala Leu
   420 425 430
   Lys Asn Leu Cys Asp Leu Gly Ile Val Asp Pro Xaa Pro Pro Xaa Cys
   435 440 445
   Asp Ile Lys Gly Ser Tyr Thr Ala Gln Phe Xaa His Thr Ile Leu Leu
   450 455 460
   Arg Pro Thr Cys Lys Glu Val Val Ser Arg Gly Asp Asp Tyr
   465 470 475
<210> 7
   <211> 478
   <212> PRT
   <213> Mouse MetAP2
<220>
   <221> SITE
   <222> (219)
   <223> May be any naturally occurring amino acid
<220>
   <221> SITE
   <222> (231)
   <223> May be any amino acid, except His
<220>
   <221> SITE
   <222> (251)
   <223> May be any naturally occurring amino acid
<220>
   <221> SITE
   <222> (262)
   <223> May be any naturally occurring amino acid
<220>
   <221> SITE
   <222> (328)
   <223> May be any naturally occurring amino acid
<220>
   <221> SITE
   <222> (331)
   <223> May be any naturally occurring amino acid
<220>
   <221> SITE
   <222> (338)..(339)
   <223> May be any naturally occurring amino acid
<220>
   <221> SITE
   <222> (364)
   <223> May be any naturally occurring amino acid
<220>
   <221> SITE
   <222> (444)
   <223> May be any naturally occurring amino acid
<220>
   <221> SITE
   <222> (447)
   <223> May be any naturally occurring amino acid
<220>
   <221> SITE
   <222> (459)
   <223> May be any naturally occurring amino acid
<400> 7
   Met Ala Gly Val Glu Gln Ala Ala Ser Phe Gly Gly His Leu Asn Gly
    1               5                  10                  15
   Asp Leu Asp Pro Asp Asp Arg Glu Glu Gly Thr Ser Ser Thr Ala Glu
               20                  25                  30
   Glu Ala Ala Lys Lys Lys Arg Arg Lys Lys Lys Lys Gly Lys Gly Ala
           35                  40                  45
   Val Ser Ala Val Gln Gln Glu Leu Asp Lys Glu Ser Gly Ala Leu Val
       50                  55                  60
   Asp Glu Val Ala Lys Gln Leu Glu Ser Gln Ala Leu Glu Glu Lys Glu
   65                  70                  75                  80
   Arg Asp Asp Asp Asp Glu Asp Gly Asp Gly Asp Ala Asp Gly Ala Thr
                       85                  90                  95
   Gly Lys Lys Lys Lys Lys Lys Lys Lys Lys Arg Gly Pro Lys Val Gln
               100                 105                 110
   Thr Asp Pro Pro Ser Val Pro Ile Cys Asp Leu Tyr Pro Asn Gly Val
           115                 120                 125
   Phe Pro Lys Gly Gln Glu Cys Glu Tyr Pro Pro Thr Gln Asp Gly Arg
       130                 135                 140
   Thr Ala Ala Trp Arg Thr Thr Ser Glu Glu Lys Lys Ala Leu Asp Gln
   145                 150                 155                 160
   Ala Ser Glu Glu Ile Trp Asn Asp Phe Arg Glu Ala Ala Glu Ala His
                   165                 170                 175
   Arg Gln Val Arg Lys Tyr Val Met Ser Trp Ile Lys Pro Gly Met Thr
               180                 185                 190
   Met Ile Glu Ile Cys Glu Lys Leu Glu Asp Cys Ser Arg Lys Leu Ile
           195                 200                 205
   Lys Glu Asn Gly Leu Asn Ala Gly Leu Ala Xaa Pro Thr Gly Cys Ser
       210                 215                 220
   Leu Asn Asn Cys Ala Ala Xaa Tyr Thr Pro Asn Ala Gly Asp Thr Thr
   225                 230                 235                 240
   Val Leu Gln Tyr Asp Asp Ile Cys Lys Ile Xaa Phe Gly Thr His Ile
                   245                 250                 255
   Ser Gly Arg Ile Ile Xaa Cys Ala Phe Thr Val Thr Phe Asn Pro Lys
               260                 265                 270
   Tyr Asp Ile Leu Leu Thr Ala Val Lys Asp Ala Thr Asn Thr Gly Ile
           275                 280                 285
   Lys Cys Ala Gly Ile Asp Val Arg Leu Cys Asp Val Gly Glu Ala Ile
       290                 295                 300
   Gln Glu Val Met Glu Ser Tyr Glu Val Glu Ile Asp Gly Lys Thr Tyr
   305                 310                 315                 320
   Gln Val Lys Pro Ile Arg Asn Xaa Asn Gly Xaa Ser Ile Gly Pro Tyr
                   325                 330                 335
   Arg Xaa Xaa Ala Gly Lys Thr Val Pro Ile Val Lys Gly Gly Glu Ala
               340                 345                 350
   Thr Arg Met Glu Glu Gly Glu Val Tyr Ala Ile Xaa Thr Phe Gly Ser
           355                 360                 365
   Thr Gly Lys Gly Val Val His Asp Asp Met Glu Cys Ser His Tyr Met
       370                 375                 380
   Lys Asn Phe Asp Val Gly His Val Pro Ile Arg Leu Pro Arg Thr Lys
   385                 390                 395                 400
   His Leu Leu Asn Val Ile Asn Glu Asn Phe Gly Thr Leu Ala Phe Cys
                   405                 410                 415
   Arg Arg Trp Leu Asp Arg Leu Gly Glu Ser Lys Tyr Leu Met Ala Leu
               420                 425                 430
   Lys Asn Leu Cys Asp Leu Gly Ile Val Asp Pro Xaa Pro Pro Xaa Cys
             435                 440                 445
   Asp Ile Lys Gly Ser Tyr Thr Ala Gln Phe Xaa His Thr Ile Leu Leu
       450                 455                 460
   Arg Pro Thr Cys Lys Glu Val Val Ser Arg Gly Asp Asp Tyr
   465                 470                 475
<210> 8
   <211> 421
   <212> PRT
   <213> Yeast MetAP2
<220>
   <221> SITE
   <222> (162)
   <223> May be any naturally occurring amino acid
<220>
   <221> SITE
   <222> (174)
   <223> May be any amino acid, except His
<220>
   <221> SITE
   <222> (194)
   <223> May be any naturally occurring amino acid
<220>
   <221> SITE
   <222> (205)
   <223> May be any naturally occurring amino acid
<220>
   <221> SITE
   <222> (271)
   <223> May be any naturally occurring amino acid
<220>
   <221> SITE
   <222> (274)
   <223> May be any naturally occurring amino acid
<220>
   <221> SITE
   <222> (281)..(282)
   <223> May be any naturally occurring amino acid
<220>
   <221> SITE
   <222> (307)
   <223> May be any naturally occurring amino acid
<220>
   <221> SITE
   <222> (387)
   <223> May be any naturally occurring amino acid
<220>
   <221> SITE
   <222> (390)
   <223> May be any naturally occurring amino acid
<220>
   <221> SITE
   <222> (402)
   <223> May be any naturally occurring amino acid
<400> 8
   Met Thr Asp Ala Glu Ile Glu Asn Ser Pro Ala Ser Asp Leu Lys Glu
   1               5                   10                  15
   Leu Asn Leu Glu Asn Glu Gly Val Glu Gln Gln Asp Gln Ala Lys Ala
               20                  25                  30
   Asp Glu Ser Asp Pro Val Glu Ser Lys Lys Lys Lys Asn Lys Lys Lys
           35                  40                  45
   Lys Lys Lys Lys Ser Asn Val Lys Lys Ile Glu Leu Leu Phe Pro Asp
       50                  55                  60
   Gly Lys Tyr Pro Glu Gly Ala Trp Met Asp Tyr His Gln Asp Phe Asn
   65                  70                  75                  80
   Leu Gln Arg Thr Thr Asp Glu Glu Ser Arg Tyr Leu Lys Arg Asp Leu
                   85                  90                  95
   Glu Arg Ala Glu His Trp Asn Asp Val Arg Lys Gly Ala Glu Ile His
               100                 105                 110
   Arg Arg Val Arg Arg Ala Ile Lys Asp Arg Ile Val Pro Gly Met Lys
           115                 120                 125
   Leu Met Asp Ile Ala Asp Met Ile Glu Asn Thr Thr Arg Lys Tyr Thr
       130                 135                 140
   Gly Ala Glu Asn Leu Leu Ala Met Glu Asp Pro Lys Ser Gln Gly Ile
   145                 150                 155                 160
   Gly Xaa Pro Thr Gly Leu Ser Leu Asn His Cys Ala Ala Xaa Phe Thr
                   165                 170                 175
   Pro Asn Ala Gly Asp Lys Thr Val Leu Lys Tyr Glu Asp Val Met Lys
               180                 185                 190
   Val Xaa Tyr Gly Val Gln Val Asn Gly Asn Ile Ile Xaa Ser Ala Phe
           195                 200                 205
   Thr Val Ser Phe Asp Pro Gln Tyr Asp Asn Leu Leu Ala Ala Val Lys
       210                 215                 220
   Asp Ala Thr Tyr Thr Gly Ile Lys Glu Ala Gly Ile Asp Val Arg Leu
   225                 230                 235                 240
   Thr Asp Ile Gly Glu Ala Ile Gln Glu Val Met Glu Ser Tyr Glu Val
                   245                 250                 255
   Glu Ile Asn Gly Glu Thr Tyr Gln Val Lys Pro Cys Arg Asn Xaa Cys
               260                 265                 270
   Gly Xaa Ser Ile Ala Pro Tyr Arg Xaa Xaa Gly Gly Lys Ser Val Pro
           275                 280                 285
   Ile Val Lys Asn Gly Asp Thr Thr Lys Met Glu Glu Gly Glu His Phe
       290                 295                 300
   Ala Ile Xaa Thr Phe Gly Ser Thr Gly Arg Gly Tyr Val Thr Ala Gly
   305                 310                 315                 320
   Gly Glu Val Ser His Tyr Ala Arg Ser Ala Glu Asp His Gln Val Met
   325 330 335
   Pro Thr Leu Asp Ser Ala Lys Asn Leu Leu Lys Thr Ile Asp Arg Asn
   340 345 350
   Phe Gly Thr Leu Pro Phe Cys Arg Arg Tyr Leu Asp Arg Leu Gly Gln
   355 360 365
   Glu Lys Tyr Leu Phe Ala Leu Asn Asn Leu Val Arg His Gly Leu Val
   370 375 380
   Gln Asp Xaa Pro Pro Xaa Asn Asp Ile Pro Gly Ser Tyr Thr Ala Gln
   385 390 395 400
   Phe Xaa His Thr Ile Leu Leu His Ala His Lys Lys Glu Val Val Ser
   405 410 415
   Lys Gly Asp Asp Tyr
   420
<210> 9
   <211> 1437
   <212> DNA
   <213> Human variant MetAP2
<220>
   <221> misc_feature
   <222> (693)
   <223> Any nucleotide
<400> 9
   atggcgggcg tggaggaggt agcggcctcc gggagccacc tgaatggcga cctggatcca 60
   gacgacaggg aagaaggagc tgcctctacg gctgaggaag cagccaagaa aaaaagacga 120
   aagaagaaga agagcaaagg gccttctgca gcaggggaac aggaacctga taaagaatca 180
   ggagcctcag tggatgaagt agcaagacag ttggaaagat cagcattgga agataaagaa 240
   agagatgaag atgatgaaga tggagatggc gatggagatg gagcaactgg aaagaagaag 300
   aaaaagaaga agaagaagag aggaccaaaa gttcaaacag accctccctc agttccaata 360
   tgtgacctgt atcctaatgg tgtatttccc aaaggacaag aatgcgaata cccacccaca 420
   caagatgggc gaacagctgc ttggagaact acaagtgaag aaaagaaagc attagatcag 480
   gcaagtgaag agatttggaa tgattttcga gaagctgcag aagcacatcg acaagttaga 540
   aaatacgtaa tgagctggat caagcctggg atgacaatga tagaaatctg tgaaaagttg 600
   gaagactgtt cacgcaagtt aataaaagag aatggattaa atgcaggcct ggcatttcct 660
   actggatgtt ctctcaataa ttgtgctgcc gcntatactc ccaatgccgg tgacacaaca 720
   gtattacagt atgatgacat ctgtaaaata gactttggaa cacatataag tggtaggatt 780
   attgactgtg cttttactgt cacttttaat cccaaatatg atacgttatt aaaagctgta 840
   aaagatgcta ctaacactgg aataaagtgt gctggaattg atgttcgtct gtgtgatgtt 900
   ggtgaggcca tccaagaagt tatggagtcc tatgaagttg aaatagatgg gaagacatat 960
   caagtgaaac caatccgtaa tctaaatgga cattcaattg ggcaatatag aatacatgct 1020
   ggaaaaacag tgccgattgt gaaaggaggg gaggcaacaa gaatggagga aggagaagta 1080
   tatgcaattg aaacctttgg tagtacagga aaaggtgttg ttcatgatga tatggaatgt 1140
   tcacattaca tgaaaaattt tgatgttgga catgtgccaa taaggcttcc aagaacaaaa 1200
   cacttgttaa atgtcatcaa tgaaaacttt ggaacccttg ccttctgccg cagatggctg 1260
   gatcgcttgg gagaaagtaa atacttgatg gctctgaaga atctgtgtga cttgggcatt 1320
   gtagatccat atccaccatt atgtgacatt aaaggatcat atacagcgca atttgaacat 1380
   accatcctgt tgcgtccaac atgtaaagaa gttgtcagca gaggagatga ctattaa 1437
<210> 10
   <211> 1437
   <212> DNA
   <213> Mouse variant MetAP2
<220>
   <221> misc_feature
   <222> (693)
   <223> Any nucleotide
<400> 10
   atggcgggcg tggagcaggc agcgtccttc gggggccacc tgaatggcga cctggatcca 60
   gacgacaggg aagagggaac ctccagcacg gccgaggaag ccgccaagaa gaaaagacgg 120
   aagaagaaga agggcaaagg ggctgtgtca gcagtgcaac aagaacttga taaagaatcc 180
   ggagccttgg tggatgaagt agcaaaacag ctggagagcc aagcactgga ggagaaggag 240
   agagatgacg acgatgaaga tggagatggt gatgctgatg gtgcaactgg gaagaagaag 300
   aaaaagaaga agaagaagag aggaccaaaa gttcaaacag accctccctc agttccaata 360
   tgtgacctgt atcctaatgg tgtatttccc aaaggacaag agtgtgaata cccacccaca 420
   caagatgggc ggacagctgc ttggagaacc acaagtgagg aaaaaaaggc cctagaccag 480
   gccagtgagg agatctggaa cgacttccga gaagctgcgg aggcacatcg gcaagttagg 540
   aaatatgtca tgagctggat caagcctggg atgacgatga tagaaatctg tgagaagttg 600
   gaagactgtt cccgaaagct aataaaggaa aatgggttaa atgcaggcct ggcgttcccc 660
   actgggtgtt ctctcaacaa ctgtgctgcc gcntacactc ccaatgctgg tgacacgaca 720
   gtcttgcagt atgatgacat ctgtaagata gactttggaa cacatataag tggtagaata 780
   atcgattgtg cttttactgt tacttttaat cccaaatatg acacactatt aacagctgta 840
   aaggatgcca ctaatactgg aataaagtgt gctgggattg acgttcgtct ctgcgatgtc 900
   ggtgaggcca ttcaagaagt tatggaatcc tatgaagtag aaatagatgg gaagacatac 960
   caagtgaaac ccatacgtaa cttaaatgga cattcaattg ggccatatag aattcacgct 1020
   ggaaaaacgg tgcccattgt gaaaggaggg gaagctacaa gaatggagga aggagaggtg 1080
   tatgccattg agacctttgg gagcacgggg aagggcgtgg ttcatgacga catggaatgt 1140
   tcacactaca tgaaaaattt tgatgtgggg cacgtgccaa taaggcttcc aagaacaaaa 1200
   cacttgttaa atgtcatcaa cgaaaacttc ggtacccttg ccttctgccg aaggtggctg 1260
   gatcgcttgg gagaaagtaa atacttaatg gctctgaaga atctgtgtga cttgggcatt 1320
   gtagatccat acccaccact gtgtgacatt aaagggtcat atacagcaca gtttgaacac 1380
   actatactgt tgcgtccaac ctgtaaagaa gttgtcagca gaggagatga ctattaa    1437
<210> 11
   <211> 1308
   <212> DNA
   <213> Yeast MetAP2 variant
<220>
   <221> misc_feature
   <222> (522)
   <223> Any nucleotide
<400> 11
   atgacagacg ctgaaataga aaattcccct gcttctgatt taaaagaatt gaatttggag 60
   aatgaaggcg ttgaacagca agaccaggca aaagctgacg agtcagaccc agtagaaagc 120
   aaaaagaaga agaacaagaa aaagaagaag aagaaaagca atgtgaagaa gattgaatta 180
   ctgtttccag atggaaagta cccagaaggt gcgtggatgg actatcatca agatttcaat 240
   ctgcaaagaa ccacggatga agaatcacgt tatttgaaaa gggatctgga aagggccgaa 300
   cattggaatg atgtcagaaa gggtgctgag atacatcgtc gtgtgagaag ggccatcaag 360
   gacagaatcg ttcctgggat gaagttaatg gatatcgctg acatgatcga aaatactaca 420
   agaaagtata caggtgccga aaatttatta gcgatggagg atcccaaatc tcaaggtatt 480
   gggtttccaa cgggtctctc tctcaaccat tgtgctgcag cnttcacacc caatgcaggc 540
   gacaaaaccg ttctgaaata cgaagacgtg atgaaggtag attatggtgt gcaggtaaac 600
   ggtaacatca ttgattctgc ctttactgtt tcctttgatc cacaatacga taacctgcta 660
   gccgctgtaa aggacgctac ttacacgggt attaaagaag cgggtatcga tgtgagatta 720
   accgacatcg gtgaagccat ccaagaagtt atggaatcct acgaagtgga aatcaatggt 780
   gagacttacc aggttaaacc ttgtcgtaat ctatgtggcc acagtatcgc accatatcgt 840
   atccacggcg gtaaatccgt tcccatcgtc aaaaatgggg acactacaaa aatggaggaa 900
   ggtgagcact ttgccattga aacttttggt tctactggta gaggttatgt tactgccggt 960
   ggggaagttt ctcattatgc cagatctgct gaagaccatc aggtaatgcc cacgttagac 1020
   agcgccaaga acttgttaaa aacgatagac cgcaactttg ggactttacc gttctgtcgc 1080
   cgatacctag acagacttgg ccaagagaaa tacttatttg cgttgaataa cttggttaga 1140
   cacggtttag tacaggatta tccaccattg aacgatatcc ccggatccta cactgcacaa 1200
   ttcgaacaca ccatcttgtt gcatgctcac aaaaaggaag tcgtttcgaa aggtgatgac 1260
   tactgaggta aaatgcgctt tcaaatggcc tcctcactag gtatatga           1308
<210> 12
   <211> 478
   <212> PRT
   <213> Human MetAP2
<400> 12
   Met Ala Gly Val Glu Glu Val Ala Ala Ser Gly Ser His Leu Asn Gly
   1               5                   10                  15
   Asp Leu Asp Pro Asp Asp Arg Glu Glu Gly Ala Ala Ser Thr Ala Glu
               20                  25                  30
   Glu Ala Ala Lys Lys Lys Arg Arg Lys Lys Lys Lys Ser Lys Gly Pro
           35                  40                  45
   Ser Ala Ala Gly Glu Gln Glu Pro Asp Lys Glu Ser Gly Ala Ser Val
       50                  55                  60
   Asp Glu Val Ala Arg Gln Leu Glu Arg Ser Ala Leu Glu Asp Lys Glu
   65                  70                  75                  80
   Arg Asp Glu Asp Asp Glu Asp Gly Asp Gly Asp Gly Asp Gly Ala Thr
                   85                  90                  95
   Gly Lys Lys Lys Lys Lys Lys Lys Lys Lys Arg Gly Pro Lys Val Gln
               100                 105                 110
   Thr Asp Pro Pro Ser Val Pro Ile Cys Asp Leu Tyr Pro Asn Gly Val
           115                 120                 125
   Phe Pro Lys Gly Gln Glu Cys Glu Tyr Pro Pro Thr Gln Asp Gly Arg
       130                 135                 140
   Thr Ala Ala Trp Arg Thr Thr Ser Glu Glu Lys Lys Ala Leu Asp Gln
   145                 150                 155                 160
   Ala Ser Glu Glu Ile Trp Asn Asp Phe Arg Glu Ala Ala Glu Ala His
                   165                 170                 175
   Arg Gln Val Arg Lys Tyr Val Met Ser Trp Ile Lys Pro Gly Met Thr
               180                 185                 190
   Met Ile Glu Ile Cys Glu Lys Leu Glu Asp Cys Ser Arg Lys Leu Ile
           195                 200                 205
   Lys Glu Asn Gly Leu Asn Ala Gly Leu Ala Phe Pro Thr Gly Cys Ser
       210                 215                 220
   Leu Asn Asn Cys Ala Ala His Tyr Thr Pro Asn Ala Gly Asp Thr Thr
   225                 230                 235                 240
   Val Leu Gln Tyr Asp Asp Ile Cys Lys Ile Asp Phe Gly Thr His Ile
                   245                 250                 255
   Ser Gly Arg Ile Ile Asp Cys Ala Phe Thr Val Thr Phe Asn Pro Lys
               260                 265                 270
   Tyr Asp Thr Leu Leu Lys Ala Val Lys Asp Ala Thr Asn Thr Gly Ile
           275                 280                 285
   Lys Cys Ala Gly Ile Asp Val Arg Leu Cys Asp Val Gly Glu Ala Ile
       290                 295                 300
   Gln Glu Val Met Glu Ser Tyr Glu Val Glu Ile Asp Gly Lys Thr Tyr
   305                 310                 315                 320
   Gln Val Lys Pro Ile Arg Asn Leu Asn Gly His Ser Ile Gly Gln Tyr
                   325                 330                 335
   Arg Ile His Ala Gly Lys Thr Val Pro Ile Val Lys Gly Gly Glu Ala
               340                 345                 350
   Thr Arg Met Glu Glu Gly Glu Val Tyr Ala Ile Glu Thr Phe Gly Ser
           355                 360                 365
   Thr Gly Lys Gly Val Val His Asp Asp Met Glu Cys Ser His Tyr Met
       370                 375                 380
   Lys Asn Phe Asp Val Gly His Val Pro Ile Arg Leu Pro Arg Thr Lys
   385                 390                 395                 400
   His Leu Leu Asn Val Ile Asn Glu Asn Phe Gly Thr Leu Ala Phe Cys
                   405                 410                 415
   Arg Arg Trp Leu Asp Arg Leu Gly Glu Ser Lys Tyr Leu Met Ala Leu
               420                 425                 430
   Lys Asn Leu Cys Asp Leu Gly Ile Val Asp Pro Tyr Pro Pro Leu Cys
           435                 440                 445
   Asp Ile Lys Gly Ser Tyr Thr Ala Gln Phe Glu His Thr Ile Leu Leu
       450                 455                 460
   Arg Pro Thr Cys Lys Glu Val Val Ser Arg Gly Asp Asp Tyr
   465                 470                 475
<210> 13
   <211> 478
   <212> PRT
   <213> Mouse MetAP2
<400> 13
   Met Ala Gly Val Glu Gln Ala Ala Ser Phe Gly Gly His Leu Asn Gly
   1               5                   10                  15
   Asp Leu Asp Pro Asp Asp Arg Glu Glu Gly Thr Ser Ser Thr Ala Glu
               20                  25                  30
   Glu Ala Ala Lys Lys Lys Arg Arg Lys Lys Lys Lys Gly Lys Gly Ala
           35                  40                  45
   Val Ser Ala Val Gln Gln Glu Leu Asp Lys Glu Ser Gly Ala Leu Val
       50                  55                  60
   Asp Glu Val Ala Lys Gln Leu Glu Ser Gln Ala Leu Glu Glu Lys Glu
   65                  70                  75                  80
   Arg Asp Asp Asp Asp Glu Asp Gly Asp Gly Asp Ala Asp Gly Ala Thr
                   85                  90                  95
   Gly Lys Lys Lys Lys Lys Lys Lys Lys Lys Arg Gly Pro Lys Val Gln
               100                 105                 110
   Thr Asp Pro Pro Ser Val Pro Ile Cys Asp Leu Tyr Pro Asn Gly Val
           115                 120                 125
   Phe Pro Lys Gly Gln Glu Cys Glu Tyr Pro Pro Thr Gln Asp Gly Arg
       130                 135                 140
   Thr Ala Ala Trp Arg Thr Thr Ser Glu Glu Lys Lys Ala Leu Asp Gln
   145                 150                 155                 160
   Ala Ser Glu Glu Ile Trp Asn Asp Phe Arg Glu Ala Ala Glu Ala His
                   165                 170                 175
   Arg Gln Val Arg Lys Tyr Val Met Ser Trp Ile Lys Pro Gly Met Thr
   180 185 190
   Met Ile Glu Ile Cys Glu Lys Leu Glu Asp Cys Ser Arg Lys Leu Ile
   195 200 205
   Lys Glu Asn Gly Leu Asn Ala Gly Leu Ala Phe Pro Thr Gly Cys Ser
   210 215 220
   Leu Asn Asn Cys Ala Ala His Tyr Thr Pro Asn Ala Gly Asp Thr Thr
   225 230 235 240
   Val Leu Gln Tyr Asp Asp Ile Cys Lys Ile Asp Phe Gly Thr His Ile
   245 250 255
   Ser Gly Arg Ile Ile Asp Cys Ala Phe Thr Val Thr Phe Asn Pro Lys
   260 265 270
   Tyr Asp Ile Leu Leu Thr Ala Val Lys Asp Ala Thr Asn Thr Gly Ile
   275 280 285
   Lys Cys Ala Gly Ile Asp Val Arg Leu Cys Asp Val Gly Glu Ala Ile
   290 295 300
   Gln Glu Val Met Glu Ser Tyr Glu Val Glu Ile Asp Gly Lys Thr Tyr
   305 310 315 320
   Gln Val Lys Pro Ile Arg Asn Leu Asn Gly His Ser Ile Gly Pro Tyr
   325 330 335
   Arg Ile His Ala Gly Lys Thr Val Pro Ile Val Lys Gly Gly Glu Ala
   340 345 350
   Thr Arg Met Glu Glu Gly Glu Val Tyr Ala Ile Glu Thr Phe Gly Ser
   355 360 365
   Thr Gly Lys Gly Val Val His Asp Asp Met Glu Cys Ser His Tyr Met
   370 375 380
   Lys Asn Phe Asp Val Gly His Val Pro Ile Arg Leu Pro Arg Thr Lys
   385 390 395 400
   His Leu Leu Asn Val Ile Asn Glu Asn Phe Gly Thr Leu Ala Phe Cys
   405 410 415
   Arg Arg Trp Leu Asp Arg Leu Gly Glu Ser Lys Tyr Leu Met Ala Leu
   420 425 430
   Lys Asn Leu Cys Asp Leu Gly Ile Val Asp Pro Tyr Pro Pro Leu Cys
   435 440 445
   Asp Ile Lys Gly Ser Tyr Thr Ala Gln Phe Glu His Thr Ile Leu Leu
   450 455 460
   Arg Pro Thr Cys Lys Glu Val Val Ser Arg Gly Asp Asp Tyr
   465 470 475
<210> 14
   <211> 437
   <212> PRT
   <213> Yeast MetAP2
<400> 14
   Met Thr Asp Ala Glu Ile Glu Asn Ser Pro Ala Ser Asp Leu Lys Glu
   1 5 10 15
   Leu Asn Leu Glu Asn Glu Gly Val Glu Gln Gln Asp Gln Ala Lys Ala
   20 25 30
   Asp Glu Ser Asp Pro Val Glu Ser Lys Lys Lys Lys Asn Lys Lys Lys
   35 40 45
   Lys Lys Lys Lys Ser Asn Val Lys Lys Ile Glu Leu Leu Phe Pro Asp
   50 55 60
   Gly Lys Tyr Pro Glu Gly Ala Trp Met Asp Tyr His Gln Asp Phe Asn
   65 70 75 80
   Leu Gln Arg Thr Thr Asp Glu Glu Ser Arg Tyr Leu Lys Arg Asp Leu
   85 90 95
   Glu Arg Ala Glu His Trp Asn Asp Val Arg Lys Gly Ala Glu Ile His
   100 105 110
   Arg Arg Val Arg Arg Ala Ile Lys Asp Arg Ile Val Pro Gly Met Lys
   115 120 125
   Leu Met Asp Ile Ala Asp Met Ile Glu Asn Thr Thr Arg Lys Tyr Thr
   130 135 140
   Gly Ala Glu Asn Leu Leu Ala Met Glu Asp Pro Lys Ser Gln Gly Ile
   145 150 155 160
   Gly Phe Pro Thr Gly Leu Ser Leu Asn His Cys Ala Ala His Phe Thr
   165 170 175
   Pro Asn Ala Gly Asp Lys Thr Val Leu Lys Tyr Glu Asp Val Met Lys
   180 185 190
   Val Asp Tyr Gly Val Gln Val Asn Gly Asn Ile Ile Asp Ser Ala Phe
   195 200 205
   Thr Val Ser Phe Asp Pro Gln Tyr Asp Asn Leu Leu Ala Ala Val Lys
   210 215 220
   Asp Ala Thr Tyr Thr Gly Ile Lys Glu Ala Gly Ile Asp Val Arg Leu
   225 230 235 240
   Thr Asp Ile Gly Glu Ala Ile Gln Glu Val Met Glu Ser Tyr Glu Val
   245 250 255
   Glu Ile Asn Gly Glu Thr Tyr Gln Val Lys Pro Cys Arg Asn Leu Cys
   260 265 270
   Gly His Ser Ile Ala Pro Tyr Arg Ile His Gly Gly Lys Ser Val Pro
   275 280 285
   Ile Val Lys Asn Gly Asp Thr Thr Lys Met Glu Glu Gly Glu His Phe
   290 295 300
   Ala Ile Glu Thr Phe Gly Ser Thr Gly Arg Gly Tyr Val Thr Ala Gly
   305 310 315 320
   Gly Glu Val Ser His Tyr Ala Arg Ser Ala Glu Asp His Gln Val Met
   325 330 335
   Pro Thr Leu Asp Ser Ala Lys Asn Leu Leu Lys Thr Ile Asp Arg Asn
   340 345 350
   Phe Gly Thr Leu Pro Phe Cys Arg Arg Tyr Leu Asp Arg Leu Gly Gln
   355 360 365
   Glu Lys Tyr Leu Phe Ala Leu Asn Asn Leu Val Arg His Gly Leu Val
   370 375 380
   Gln Asp Tyr Pro Pro Leu Asn Asp Ile Pro Gly Ser Tyr Thr Ala Gln
   385 390 395 400
   Phe Glu His Thr Ile Leu Leu His Ala His Lys Lys Glu Val Val Ser
   405 410 415
   Lys Gly Asp Asp Tyr Gly Lys Met Arg Phe Gln Met Ala Ser Ser Leu
   420 425 430
   Gly Ile Ile Leu Leu
   435
<210> 15
   <211> 71
   <212> PRT
   <213> Rat polylysine
<400> 15
   Lys Lys Lys Arg Arg Lys Lys Lys Lys Gly Lys Gly Ala Val Ser Ala
   1 5 10 15
   Gly Gln Gln Glu Leu Asp Lys Glu Ser Gly Thr Ser Val Asp Glu Val
   20 25 30
   Ala Lys Gln Leu Glu Arg Gln Ala Leu Glu Glu Lys Glu Lys Asp Asp
   35 40 45
   Asp Asp Glu Asp Gly Asp Gly Asp Gly Asp Gly Ala Ala Gly Lys Lys
   50 55 60
   Lys Lys Lys Lys Lys Lys Lys
   65 70
<210> 16
   <211> 480
   <212> PRT
   <213> Rat dnvMetAP2
<220>
   <221> SITE
   <222> (219)
   <223> May be any naturally occurring amino acid
<220>
   <221> SITE
   <222> (231)
   <223> May be any amino acid, except His
<220>
   <221> SITE
   <222> (251)
   <223> May be any naturally occurring amino acid
<220>
   <221> SITE
   <222> (262)
   <223> May be any naturally occurring amino acid
<220>
   <221> SITE
   <222> (328)
   <223> May be any naturally occurring amino acid
<220>
   <221> SITE
   <222> (331)
   <223> May be any naturally occurring amino acid
<220>
   <221> SITE
   <222> (338)..(339)
   <223> May be any naturally occurring amino acid
<220>
   <221> SITE
   <222> (364)
   <223> May be any naturally occurring amino acid
<220>
   <221> SITE
   <222> (444)
   <223> May be any naturally occurring amino acid
<220>
   <221> SITE
   <222> (447)
   <223> May be any naturally occurring amino acid
<220>
   <221> SITE
   <222> (459)
   <223> May be any naturally occurring amino acid
<400> 16
   Met Ala Gly Val Glu Glu Ala Ser Ser Phe Gly Gly His Leu Asn Arg
   1               5                   10                  15
   Asp Leu Asp Pro Asp Asp Arg Glu Glu Gly Thr Ser Ser Thr Ala Glu
               20                  25                  30
   Glu Ala Ala Lys Lys Lys Arg Arg Lys Lys Lys Lys Gly Lys Gly Ala
           35                  40                  45
   Val Ser Ala Gly Gln Gln Glu Leu Asp Lys Glu Ser Gly Thr Ser Val
       50                  55                  60
   Asp Glu Val Ala Lys Gln Leu Glu Arg Gln Ala Leu Glu Glu Lys Glu
   65                  70                  75                  80
   Lys Asp Asp Asp Asp Glu Asp Gly Asp Gly Asp Gly Asp Gly Ala Ala
                   85                  90                  95
   Gly Lys Lys Lys Lys Lys Lys Lys Lys Lys Arg Gly Pro Arg Val Gln
               100                 105                 110
   Thr Asp Pro Pro Ser Val Pro Ile Cys Asp Leu Tyr Pro Asn Gly Val
           115                 120                 125
   Phe Pro Lys Gly Gln Glu Cys Glu Tyr Pro Pro Thr Gln Asp Gly Arg
       130                 135                 140
   Thr Ala Ala Trp Arg Thr Thr Ser Glu Glu Lys Lys Ala Leu Asp Gln
   145                 150                 155                 160
   Ala Ser Glu Glu Ile Trp Asn Asp Phe Arg Glu Ala Ala Glu Ala His
                   165                 170                 175
   Arg Gln Val Arg Lys Tyr Val Met Ser Trp Ile Lys Pro Gly Met Thr
               180                 185                 190
   Met Ile Glu Ile Cys Glu Lys Leu Glu Asp Cys Ser Arg Lys Leu Ile
           195                 200                 205
   Lys Glu Asn Gly Leu Asn Ala Gly Leu Ala Xaa Pro Thr Gly Cys Ser
       210                 215                 220
   Leu Asn Asn Cys Ala Ala Xaa Tyr Thr Pro Asn Ala Gly Asp Thr Thr
   225                 230                 235                 240
   Val Leu Gln Tyr Asp Asp Ile Cys Lys Ile Xaa Phe Gly Thr His Ile
                   245                 250                 255
   Ser Gly Arg Ile Ile Xaa Cys Ala Phe Thr Val Thr Phe Asn Pro Lys
               260                 265                 270
   Tyr Asp Ile Leu Leu Lys Ala Val Lys Asp Ala Thr Asn Thr Gly Ile
           275                 280                 285
   Lys Cys Ala Gly Ile Asp Val Arg Leu Cys Asp Val Gly Glu Ala Ile
       290                 295                 300
   Gln Glu Val Met Glu Ser Tyr Glu Val Glu Ile Asp Gly Lys Thr Tyr
   305                 310                 315                 320
   Gln Val Lys Pro Ile Arg Asn Xaa Asn Gly Xaa Ser Ile Gly Pro Tyr
                   325                 330                 335
   Arg Xaa Xaa Ala Gly Lys Thr Val Pro Ile Val Lys Gly Gly Glu Ala
               340                 345                 350
   Thr Arg Met Glu Glu Gly Glu Val Tyr Ala Ile Xaa Thr Phe Gly Ser
           355                 360                 365
   Thr Gly Lys Gly Val Val His Asp Asp Met Glu Cys Ser His Tyr Met
       370                 375                 380
   Lys Asn Phe Asp Val Gly His Val Pro Ile Arg Leu Pro Arg Thr Lys
   385                 390                 395                 400
   His Leu Leu Asn Val Ile Asn Glu Asn Phe Gly Thr Leu Ala Phe Cys
                   405                 410                 415
   Arg Arg Trp Leu Asp Arg Leu Gly Glu Ser Lys Tyr Leu Met Ala Leu
               420                 425                 430
   Lys Asn Leu Cys Asp Leu Gly Ile Val Asp Pro Xaa Pro Pro Xaa Cys
           435                 440                 445
   Asp Ile Lys Gly Ser Tyr Thr Ala Gln Phe Xaa His Thr Ile Leu Cys
       450                 455                 460
   Ala Gln Pro Val Lys Lys Leu Ser Ala Glu Glu Met Thr Ile Lys Thr
   465                 470                 475                 480
<210> 17
   <211> 480
   <212> PRT
   <213> Rat MetAP2
<400> 17
   Met Ala Gly Val Glu Glu Ala Ser Ser Phe Gly Gly His Leu Asn Arg
   1 5 10 15
   Asp Leu Asp Pro Asp Asp Arg Glu Glu Gly Thr Ser Ser Thr Ala Glu
   20 25 30
   Glu Ala Ala Lys Lys Lys Arg Arg Lys Lys Lys Lys Gly Lys Gly Ala
   35 40 45
   Val Ser Ala Gly Gln Gln Glu Leu Asp Lys Glu Ser Gly Thr Ser Val
   50 55 60
   Asp Glu Val Ala Lys Gln Leu Glu Arg Gln Ala Leu Glu Glu Lys Glu
   65 70 75 80
   Lys Asp Asp Asp Asp Glu Asp Gly Asp Gly Asp Gly Asp Gly Ala Ala
   85 90 95
   Gly Lys Lys Lys Lys Lys Lys Lys Lys Lys Arg Gly Pro Arg Val Gln
   100 105 110
   Thr Asp Pro Pro Ser Val Pro Ile Cys Asp Leu Tyr Pro Asn Gly Val
   115 120 125
   Phe Pro Lys Gly Gln Glu Cys Glu Tyr Pro Pro Thr Gln Asp Gly Arg
   130 135 140
   Thr Ala Ala Trp Arg Thr Thr Ser Glu Glu Lys Lys Ala Leu Asp Gln
   145 150 155 160
   Ala Ser Glu Glu Ile Trp Asn Asp Phe Arg Glu Ala Ala Glu Ala His
   165 170 175
   Arg Gln Val Arg Lys Tyr Val Met Ser Trp Ile Lys Pro Gly Met Thr
   180 185 190
   Met Ile Glu Ile Cys Glu Lys Leu Glu Asp Cys Ser Arg Lys Leu Ile
   195 200 205
   Lys Glu Asn Gly Leu Asn Ala Gly Leu Ala Phe Pro Thr Gly Cys Ser
   210 215 220
   Leu Asn Asn Cys Ala Ala His Tyr Thr Pro Asn Ala Gly Asp Thr Thr
   225 230 235 240
   Val Leu Gln Tyr Asp Asp Ile Cys Lys Ile Asp Phe Gly Thr His Ile
   245 250 255
   Ser Gly Arg Ile Ile Asp Cys Ala Phe Thr Val Thr Phe Asn Pro Lys
   260 265 270
   Tyr Asp Ile Leu Leu Lys Ala Val Lys Asp Ala Thr Asn Thr Gly Ile
   275 280 285
   Lys Cys Ala Gly Ile Asp Val Arg Leu Cys Asp Val Gly Glu Ala Ile
   290 295 300
   Gln Glu Val Met Glu Ser Tyr Glu Val Glu Ile Asp Gly Lys Thr Tyr
   305 310 315 320
   Gln Val Lys Pro Ile Arg Asn Leu Asn Gly His Ser Ile Gly Pro Tyr
   325 330 335
   Arg Ile His Ala Gly Lys Thr Val Pro Ile Val Lys Gly Gly Glu Ala
                   340                 345                 350
   Thr Arg Met Glu Glu Gly Glu Val Tyr Ala Ile Glu Thr Phe Gly Ser
               355                 360                 365
   Thr Gly Lys Gly Val Val His Asp Asp Met Glu Cys Ser His Tyr Met
       370                 375                 380
   Lys Asn Phe Asp Val Gly His Val Pro Ile Arg Leu Pro Arg Thr Lys
   385                 390                 395                 400
   His Leu Leu Asn Val Ile Asn Glu Asn Phe Gly Thr Leu Ala Phe Cys
                   405                 410                 415
   Arg Arg Trp Leu Asp Arg Leu Gly Glu Ser Lys Tyr Leu Met Ala Leu
               420                 425                 430
   Lys Asn Leu Cys Asp Leu Gly Ile Val Asp Pro Tyr Pro Pro Leu Cys
           435                 440                 445
   Asp Ile Lys Gly Ser Tyr Thr Ala Gln Phe Glu His Thr Ile Leu Cys
       450                 455                 460
   Ala Gln Pro Val Lys Lys Leu Ser Ala Glu Glu Met Thr Ile Lys Thr
   465                 470                 475                 480
<210> 18
   <211> 1944
   <212> DNA
   <213> Rat MetAP2 variant
<220>
   <221> misc_feature
   <222> (779)
   <223> Any nucleotide
<400> 18
   ggtgaagaag gagcgggccc tcgccgctcg ttctcgctcc ctctttctct ctctcttctt 60
   ctctctctct ttccctctcg ggcaacatgg cgggcgtgga agaggcatcg tctttcgggg 120
   gccacctgaa tcgcgacctg gatccagacg acagggaaga gggaacctcc agcacggccg 180
   aggaagccgc caagaagaaa agacggaaga agaagaaggg caaaggggct gtgtcagcag 240
   ggcaacaaga acttgataaa gaatcgggaa cctcagtgga cgaagtagca aaacagttgg 300
   agagacaagc actggaggag aaagagaaag atgatgacga tgaagatgga gatggtgatg 360
   gtgatggtgc agctgggaag aagaagaaaa agaagaagaa gaagagagga ccaagagttc 420
   aaacagaccc tccctcagtt ccaatatgtg acctgtatcc taatggtgta tttcccaaag 480
   gacaagagtg tgaataccca cccacccaag atgggcggac agctgcttgg agaaccacaa 540
   gtgaagagaa aaaggcgcta gaccaggcta gtgaggagat ttggaacgac ttccgagaag 600
   ctgccgaagc acaccggcaa gttaggaaat acgtcatgag ctggatcaag cctgggatga 660
   caatgataga aatatgtgag aagttggaag actgttcccg aaagctcata aaggagaatg 720
   ggttaaatgc aggcctggcc tttcccactg ggtgttctct caacaactgt gctgcagcnt 780
   acactcccaa tgctggtgac acgacagtct tacagtacga cgacatctgt aagatcgact 840
   ttggaacgca tataagtggt agaataattg attgtgcttt tactgttact tttaatccca 900
   aatatgacat attattaaaa gctgtaaaag atgccaccaa tactggaata aagtgtgcgg 960
   ggattgacgt ccgtctctgt gatgtcggcg aggccattca agaagttatg gagtcctatg 1020
   aagtggaaat agatgggaag acctaccaag tgaaacccat acgtaactta aatggacatt 1080
   caattgggcc atatagaatt catgctggaa aaacagtgcc cattgtgaaa ggaggggaag 1140
   ctacaaggat ggaggaagga gaggtgtatg ccattgagac ctttggtagc acagggaagg 1200
   gcgtggttca tgacgatatg gaatgttcac actacatgaa aaattttgat gtgggacacg 1260
   tgccaataag gcttccaaga acaaaacact tgttgaatgt catcaatgaa aactttggta 1320
   cccttgcctt ctgccgaagg tggctggatc gcttgggaga aagtaaatac ttaatggctc 1380
   tgaagaacct gtgtgacttg ggcattgtag atccatatcc accactctgt gacattaaag 1440
   gatcatacac agcacagttt gaacatacca tactctgcgc ccaacctgta aagaagttgt 1500
   cagcagagga gatgactatt aaaacttagt ccaaagccaa ctcaacgtct ttattttcta 1560
   agctttgttg gaacacatta taccacaagt aatttgcaac atgtctgttt taacagtgga 1620
   cctgtgtaat gccgttatcc atgtttaaag gagtttgatc aaagccaaac tgtctacatg 1680
   taattaacca aggaaaaggc tttcaagact ttactgttaa ctgtttctcc cgtctaggaa 1740
   atgctgtact gctcactagt taggaattac ttaaacgttt tgttttgaag acctaagaga 1800
   tgctttttgg atatttatat tgccatattc ttacttggat gctttgaatg actacataca 1860
   tccagttctg cacctatgcc ctctggtatt gctttttaac cttcctggaa tccattttct 1920
   aaaaaataaa gacattttca gatc                                       1944
<210> 19
   <211> 8
   <212> PRT
   <213> Artificial Sequence
<220>
   <223> Description of Artificial Sequence: synthetic transit peptide
<400> 19
   Gly Arg Lys Lys Arg Arg Gln Arg
    1               5
<210> 20
   <211> 40
   <212> DNA
   <213> Artificial Sequence
<220>
   <223> Description of Artificial Sequence: synthetic oligonucleotide
<400> 20
   gcgcaagctt atgattgaat tactgtttcc agatggaaag 40
<210> 21
   <211> 35
   <212> DNA
   <213> Artificial Sequence
<220>
   <223> Description of Artificial Sequence: synthetic oligonucleotide
<400> 21
   gcgcctcgag tcagtagtca tcacctttcg aaacg 35
<210> 22
   <211> 11
   <212> PRT
   <213> Artificial Sequence
<220>
   <223> Description of Artificial Sequence: synthetic peptide
<400> 22
   Cys Lys Glu Val Val Ser Lys Gly Asp Asp Tyr
   1               5                   10
<210> 23
   <211> 9
   <212> PRT
   <213> Artificial Sequence
<220>
   <223> Description of Artificial Sequence: synthetic peptide
<400> 23
   Tyr Pro Tyr Asp Val Pro Asp Tyr Ala
   1               5
<210> 24
   <211> 4
   <212> PRT
   <213> Artificial Sequence
<220>
   <223> Description of Artificial Sequence: synthetic peptide
<400> 24
   Met Gly Met Met
   1
<210> 25
   <211> 63
   <212> DNA
   <213> Artificial Sequence
<220>
   <223> Description of Artificial Sequence: synthetic oligonucleotide
<400> 25
   cacactcgac cgcgatgtac tactactact actactacta ctactacggg ccagatatac 60
   gcg                                                               63
<210> 26
   <211> 37
   <212> DNA
   <213> Artificial Sequence
<220>
   <223> Description of Artificial Sequence: synthetic oligonucleotide
<400> 26
   cacagaattc cccgcatccc cagcatgcct gctattg 37